# EUROPEAN PATENT APPLICATION

(11) **EP 4 671 272 A1**
(43) Date of publication of application: **31.12.2025**
(21) Application number: 24923340.4
(22) Date of filing: 27.09.2024
(51) Int. Cl.: C07K 16/18, C07K 14/47, C07K 19/00, C12N 15/13, C12N 15/12, A61P 35/04, A61P 11/00

(54) **ANTI-S100A8/A9 ANTIBODY AND USE THEREOF**

(30) Priority: 07.02.2024 CN 202410175455
(71) Applicant: Beijing Sungen Biomedical Technology Co. Ltd., Beijing 102609 (CN)
(72) Inventor: LI, Yulin, Beijing 102609 (CN); SUN, Zhiwei, Beijing 102609 (CN); LIU, Yan, Beijing 102609 (CN); ZHAO, Guangzhen, Beijing 102609 (CN); LI, Guoqi, Beijing 102609 (CN); DU, Jie, Beijing 102609 (CN); GAO, Shijuan, Beijing 102609 (CN); ZHOU, Jianhua, Beijing 102609 (CN); SHAO, Yihui, Beijing 102609 (CN); LIU, Si, Beijing 102609 (CN); LIN, Changqing, Beijing 102609 (CN)
(74) Representative: Wichmann, Hendrik
(86) International application number: PCT/CN2024/121999
(87) International publication number: WO 2025/167135

(57) **Abstract**

The present application provides an anti-S100A8/A9 antibody and use thereof. The antibody or antigen-binding fragment thereof, and mutants thereof, provided by the present application, can block the biological functions of S100A8/A9. The antibody or antigen-binding fragment thereof provided by the present application is expected to be developed into a clinical drug, with the aim of reducing the myocardial infarct size in acute myocardial infarction (AMI) patients, lowering the incidence of heart failure, and providing lifelong benefit to the AMI patients. The present application has significant clinical implications and development value.

## Description

### CROSS-REFERENCE

This application claims priority to Chinese Patent Application No. 202410175455.0, titled "Antibody Binding to S100A8 and S100A9 Heterodimers or Heterotetramers and Use Thereof", filed on February 7, 2024, which is incorporated herein by reference in its entirety.

### FIELD OF TECHNOLOGY

The present application relates to the field of molecular biology, and in particular, to a fully human antibody or an antigen-binding fragment thereof and use thereof.

### BACKGROUND

Cardiovascular diseases (CVDs) are the leading cause of death in humans and rank first in the global burden of disease. In China, 2 out of every 5 deaths are caused by CVDs, with over half being coronary heart disease (CHD)-related. Acute myocardial infarction (AMI), a severe manifestation of CHD, occurs when acute and sustained ischemia and hypoxia in coronary arteries induce cardiomyocyte necrosis, serving as a primary cause of death and disability. Current therapeutic methods prioritize rapid revascularization of the infarct-related artery to restore myocardial blood perfusion. Thrombolysis, percutaneous coronary intervention (PCI), or thrombolysis-facilitated PCI is selected based on patient status. While the widespread use of PCI surgery has improved AMI therapy and reperfusion effectively prevents cardiomyocytes from further necrosis, there are still two problems: first, the average hospital arrival time for AMI patients after onset is 5.5 hours, so recanalization of occluded blood vessels generally occurs 6 hours after infarction. During this period, cardiomyocytes have necrosed due to ischemia and hypoxia, but no early therapeutic drugs exist to reduce the myocardial infarct size. Second, ischemia-reperfusion (I/R) injury is also induced after vascular recanalization, and some cardiomyocytes have necrosed before recanalization. Cardiac dysfunction or even heart failure is still a prominent problem, and current clinical pharmacotherapies fail to ameliorate this situation.

AMI-induced cardiac injury results from synergistic cellular and pathological processes. For example, both AMI and I/R injury trigger massive inflammatory cell infiltration, and in animal experiments, inflammation suppression can reduce post-infarct damage and improve the cardiac function. However, multiple clinical trials of anti-inflammatory therapies for patients with myocardial infarction (MI) have failed, indicating the broad-spectrum inflammation suppression is inadvisable in the therapy for ischemic heart disease. This necessitates the development of targets that are more specific and targeted.

S100A8 and S100A9 (also termed MRP8 and MRP14) belong to the calcium-binding protein S100 family. Human S100A8 comprises 98 amino acids and has a molecular weight of 10.8 kD, and S100A9 comprises 113 amino acids and has a molecular weight of 13.2 kD. They both can form heterodimers, heterotetramers, and oligomers, mainly in the form of dimers, while their respective molecules can also form homologous polymers. Due to the poor stability of homodimers, they usually exert biological functions in the form of heterodimers or heterotetramers under the pathophysiological state. S100A8/S100A9 is mainly expressed by neutrophils, accounting for approximately 40% of their cytoplasmic proteins. When inflammatory cells are stressed, S100A8/S100A9 is released as alarmins that act on Toll-like receptor 4 (TLR4) and receptor for advanced glycation end products (RAGE) to regulate immune-inflammatory responses.

Our prior study *(*Circulation, 2019, 140(9):751-764) found via transcriptome sequencing that after mouse myocardial ischemia, genes related to inflammatory responses change most significantly in the early stage, and S100A8 and S100A9 among the inflammatory-related differential genes exhibit the most significant elevation, peaking at 6 hours after I/R injury, then gradually decreasing, and substantially restoring to a basal level by day 7. Subsequent studies by multiple teams *(*Circulation, 2020, 141(13):1080-1094; Eur Heart J, 2019, 40(32):2713-2723) validated these findings: S100A8 and S100A9 are massively released in the early stage of MI. Meanwhile, it is further observed that the serum S100A8/A9 levels in AMI patients 1 day post-PCI exhibit a significant elevation, and the elevation of S100A8/A9 levels correlates with the major adverse cardiovascular event (MACE) incidence and is an independent predictor of long-term MACE incidence of AMI patients *(*Circulation, 2019, 140(9):751-764). In addition, it is further validated that S100A8/A9 acts on cardiomyocyte TLR4 to regulate mitochondrial dysfunction so as to mediate myocardial injury *(*Circulation, 2019, 140(9):751-764). S100A9 gene knockout and S100A9 neutralizing antibodies or inhibitors can all attenuate cardiac injury and adverse remodeling in mice after MI or myocardial I/R, thereby improving the cardiac function *(*Circulation, 2019, 140(9):751-764; Circulation, 2020, 141(13):1080-1094; Eur Heart J, 2019, 40(32):2713-2723). The above all verify that S100A8/A9 is a superior target for early intervention therapy for MI and myocardial I/R injury and is more specific and effective than broad-spectrum anti-inflammatory therapies.

However, use of S100A8/A9 target in clinical therapy still faces many problems to be solved: first, the homology between human and mouse S100A8/A9 is low, neutralizing antibodies for mice used in previous studies are difficult to be used in the human body, and existing commercial S100A9 antibodies are not fully human or humanized monoclonal antibodies. Second, S100A8/A9 exerts an effect that causes cardiac injury in the form of heterodimers or heterotetramers. Existing S100A9-targeted neutralizing antibodies cannot specifically target S100A8/A9 dimers and tetramers. Thus, it is necessary to develop neutralizing antibodies for human S100A8/A9 and validate *in-vivo* therapeutic efficacy.

Please noted that the methods described in this section are not necessarily prior methods conceived or implemented. Unless otherwise indicated, it should not be assumed that any method described in this section is construed as prior art merely by inclusion in this section. Similarly, unless otherwise indicated, problems mentioned in this section should not be considered as recognized in any prior art.

### SUMMARY

To solve the above technical problems, the present application provides a fully human antibody or an antigen-binding fragment thereof, wherein complementarity-determining regions (CDRs) thereof defined according to a KABAT system have the following amino acid sequences: an amino acid sequence of heavy chain CDR1 comprises GX₁TX₂SX₃X₄X₅X₆X₇, wherein X₁ is F or Y, X₂ is F, H, Y, or W, X₃ is S or D, X₄ is Y, H, or F, X₅ is A or V, X₆ is M, I, or L, and X₇ is S or A; an amino acid sequence of heavy chain CDR2 comprises AISGX₈GGSX₉X₁₀YX₁₁X₁₂SVKG, wherein X₈ is S or H, X₉ is T or H, X₁₀ is Y, W, or F, X₁₁ is A, H, V, or L, and X₁₂ is D, A, or H; an amino acid sequence of heavy chain CDR3 comprises KX₁₃RPX₁₄RX₁₅X₁₆DX₁₇, wherein X₁₃ is Y, F, or H, X₁₄ is S, T, or H, X₁₅ is V or A, X₁₆ is F, Y, H, or W, and X₁₇ is S, A, F, C, H, or Y; an amino acid sequence of light chain CDR1 comprises SGDALX₁₈DKX₁₉X₂₀X₂₁, wherein X₁₈ is G or H, X₁₉ is Y or F, X₂₀ is A or M, and X₂₁ is S or A; an amino acid sequence of light chain CDR2 comprises X₂₂X₂₃X₂₄X₂₅RX₂₆X₂₇, wherein X₂₂ is E or Q, X₂₃ is D, H, or Q, X₂₄ is S, A, or H, X₂₅ is K, H, R, P, or N, X₂₆ is P, Q, D, or N, and X₂₇ is S, D, Y, or W; and an amino acid sequence of light chain CDR3 comprises X₂₈SNDX₂₉DX₃₀X₃₁W, wherein X₂₈ is Q or D, X₂₉ is A, V, I, or L, X₃₀ is S or A, and X₃₁ is V, Y, W, or R.

According to one embodiment of the present application, further provided is a polynucleotide, wherein the polynucleotide encodes the antibody or antigen-binding fragment thereof of the present application.

According to one embodiment of the present application, further provided is a recombinant vector, wherein the recombinant vector comprises the polynucleotide of the present application.

According to one embodiment of the present application, further provided is a host cell, wherein the host cell comprises the polynucleotide of the present application and/or the recombinant vector of the present application.

According to one embodiment of the present application, further provided is a pharmaceutical composition, wherein the pharmaceutical composition comprises the antibody or antigen-binding fragment thereof of the present application.

According to one embodiment of the present application, further provided is a kit, wherein the kit comprises the antibody or antigen-binding fragment thereof of the present application.

According to one embodiment of the present application, further provided is a multispecific antibody, wherein the multispecific antibody comprises the antibody or antigen-binding fragment thereof of the present application, and further comprising one or more second antibodies or antigen-binding fragments thereof which bind specifically to other antigens.

According to one embodiment of the present application, further provided is an antibody conjugate, wherein the antibody conjugate comprises the antibody or antigen-binding fragment thereof of the present application, and a second functional structure selected from an Fc, a radioisotope, a half-life extending structural moiety, a detectable label, and a drug.

According to one embodiment of the present application, further provided is use of the antibody or antigen-binding fragment thereof of the present application, the multispecific antibody of the present application, and/or the antibody conjugate of the present application in preparing a drug, a reagent, or a kit for detecting, alleviating, preventing, or treating a disease.

According to one embodiment of the present application, further provided is use of the antibody or antigen-binding fragment thereof of the present application, the multispecific antibody of the present application, and/or the antibody conjugate of the present application in preparing a drug, a reagent, or a kit for reducing a myocardial infarct size, improving a cardiac function, or improving a microcirculatory function.

According to one embodiment of the present application, further provided is a method for detecting, alleviating, preventing, or treating a disease, wherein the method comprises administrating to a subject in need thereof the antibody or antigen-binding fragment thereof of the present application, the pharmaceutical composition of the present application, the kit of the present application, the multispecific antibody of the present application, and/or the antibody conjugate of the present application.

According to one embodiment of the present application, further provided is a method for reducing a myocardial infarct size, improving a cardiac function, or improving a microcirculatory function, wherein the method comprises administrating to a subject in need thereof the antibody or antigen-binding fragment thereof of the present application, the pharmaceutical composition of the present application, the kit of the present application, the multispecific antibody of the present application, and/or the antibody conjugate of the present application.

The present application provides a fully human monoclonal antibody that can block the biological functions of S100A8/A9 heterodimers. In the present application, the *in-vivo* therapeutic efficacy of the antibody for ischemic heart disease is validated using established S100A8 and S100A9 humanized mouse models. For example, the use of the antibody during the acute phase of MI or I/R injury can inhibit target molecule-induced cardiac injury, reduce the myocardial infarct size and the subsequent fibrosis degree, and improve the cardiac function in experimental animals. Therefore, the antibody or the antigen-binding fragment provided by the present application is expected to be developed into a clinical drug, with the aim of reducing the myocardial infarct size in AMI patients, lowering the incidence of heart failure, and providing lifelong benefit to AMI patients. The present application has significant clinical implications and development value.

It should be understood that the content described in this section is neither intended to identify key or essential features of the embodiments of the present application nor to limit the scope of the present application. Other features of the present application will become readily apparent from the following description.

### BRIEF DESCRIPTION OF THE DRAWINGS

The accompanying drawings exemplarily illustrate embodiments and form part of the specification. They serve to explain exemplary implementations of the embodiments together with the textual description in the specification. The illustrated embodiments are exemplary only and do not limit the scope of the claims. Throughout the accompanying drawings, identical reference numerals designate similar but not necessarily identical elements.
Figure 1 shows a graphical result of binding activity of 4 candidate monoclonal antibodies to a human S100A8/A9 (hS100A8/A9) antigen in Example 2.
Figure 2 shows a graphical result of binding specificity of an anti-S100A8/A9 monoclonal antibody against hS100A8/A9 homologous family proteins in Example 2.
Figure 3 shows a graphical result of binding specificity of an anti-S100A8/A9 monoclonal antibody against unrelated proteins in Example 2.
Figure 4 shows a graphical result of detection of species specificity of an anti-S100A8/A9 monoclonal antibody in Example 2.
Figure 5A shows a graphical result of stability of an anti-S100A8/A9 monoclonal antibody in PBS in Example 2; Figure 5B shows a graphical result of stability of an anti-S100A8/A9 monoclonal antibody in C57BL/6 mouse serum (m-Serum) in Example 2.
Figure 6 shows a graphical result of epitope analysis of binding of an anti-S100A8/A9 monoclonal antibody to hS100A8/A9 in Example 3.
Figure 7 shows a graphical result of inhibitory effects of an anti-S100A8/A9 monoclonal antibody on inflammatory cytokine release from hS100A8/A9-stimulated human PBMCs in Example 4. Figure 7A shows a graphical result of inhibitory effects of an anti-S100A8/A9 monoclonal antibody on IL-6 release from hS100A8/A9-stimulated human PBMCs in Example 4. Figure 7B shows a graphical result of inhibitory effects of an anti-S100A8/A9 monoclonal antibody on TNFα release from hS100A8/A9-stimulated human PBMCs in Example 4.
Figure 8 shows a graphical result of proliferative toxicity of an anti-S100A8/A9 monoclonal antibody toward mouse lymphoma EL-4 cells in Example 4.
Figure 9 shows a graphical result of blocking of binding of hS100A8/A9 to engineered cell line surface TLR4 (Figure 9A) or RAGE (Figure 9B) by an anti-S100A8/A9 monoclonal antibody in Example 5.
Figure 10 shows a graphical result of blocking of binding of hS100A8/A9 to PBMCs (Figure 10A) and THP-1 cells (Figure 10B) by an anti-S100A8/A9 monoclonal antibody in Example 5.
Figure 11 shows a graphical result of *in-vitro* bioactivity of genetically engineered single-point mutants of an anti-S100A8/A9 monoclonal antibody in Example 6.2.
Figure 12 (Figure 12A and Figure 12B) shows a graphical result of *in-vitro* bioactivity of genetically engineered combinatorial mutants of an anti-S100A8/A9 monoclonal antibody in Example 6.4.
Figure 13 shows a graphical result of metabolism of an anti-S100A8/A9 monoclonal antibody in mice in Example 7.
Figure 14 shows a graphical result of effects of an anti-S100A8/A9 monoclonal antibody at low dose, moderate dose, and high dose on left ventricular ejection fraction (LVEF) in S100A8/A9 humanized mice after MI in Example 8.
Figure 15 shows a graphical result of effects of an anti-S100A8/A9 monoclonal antibody at low dose, moderate dose, and high dose on cardiac infarct size in S100A8/A9 humanized mice after MI in Example 8.
Figure 16 shows a graphical result of effects of administration of an anti-S100A8/A9 monoclonal antibody at 2, 4, 8, and 10 hours after surgery on LVEF in S100A8/A9 humanized mice after MI in Example 8.
Figure 17 shows a graphical result of effects of administration of an anti-S100A8/A9 monoclonal antibody at 2, 4, 8, and 10 hours after surgery on cardiac infarct size in S100A8/A9 humanized mice after MI in Example 8.
Figure 18 shows a graphical result of effects of an anti-S100A8/A9 monoclonal antibody on LVEF in S100A8/A9 humanized mice after myocardial I/R in Example 8.

### DESCRIPTION OF THE EMBODIMENTS

Unless otherwise indicated, all numerical values expressing quantity, concentration, ratio, mass, volume, time, temperature, thickness, technical effects, and the like used in the specification and claims shall be construed as modified by the terms "about" or "approximately" in all instances. Accordingly, unless indicated to the contrary, the numerical parameters set forth in the following specification and appended claims are approximations. For those skilled in the art, they may vary depending upon the desired properties and effects sought to be obtained through the present disclosure. Each numerical parameter should be interpreted in light of the number of significant digits and standard rounding methods, or as understood by those skilled in the art.

Although it is illustrated that numerical ranges and parameters setting forth the broad scope of the present disclosure are approximations, the numerical values set forth in specific examples are reported as precisely as practicable. However, any numerical value inherently contains certain errors necessarily resulting from the standard deviations found in their respective test measurements. Every numerical range provided in the specification shall include each narrower numerical range falling within such broader numerical range as if such narrower numerical ranges were expressly written herein.

Unless otherwise specified or contradicted by context, terms or expressions used herein shall be read in conjunction with the entirety herein and as understood by those of ordinary skill in the art. Unless otherwise defined, all technical and scientific terms used herein have the same meaning as commonly understood by those of ordinary skill in the art.

As used herein, the expression "A and/or B" encompasses the following three scenarios: (1) A alone; (2) B alone; and (3) both A and B together. The expression "A, B, and/or C" encompasses the following seven scenarios: (1) A alone; (2) B alone; (3) C alone; (4) A and B together; (5) A and C together; (6) B and C together; and (7) A, B, and C together. The meaning of analogous expressions can be determined similarly.

In the present application, the terms "nucleic acid" and "polynucleotide" are used interchangeably to refer to a polymer of nucleotides of any length, including deoxyribonucleotides, ribonucleotides, combinations thereof, and analogs thereof.

In the present application, the terms "polypeptide" and "peptide" are used interchangeably to refer to a polymer of amino acids of any length. Thus, polypeptides, oligopeptides, proteins, antibodies, and enzymes all fall within the scope of the definition of polypeptide.

In the present application, the term "fragment" with respect to a sequence refers to a portion of the sequence. For example, a fragment of a nucleic acid sequence refers to a portion of the nucleic acid sequence; a fragment of an amino acid sequence refers to a portion of the amino acid sequence.

In the present application, the term "identity" with respect to sequences refers to the degree of identical residues occurring at the identical positions when two (nucleotide or amino acid) sequences are aligned, and is typically expressed as a percentage. Preferably, identity is determined over the full length of the sequences being compared. Thus, two copies with identical sequences have 100% identity. Those skilled in the art know that sequence identity may be determined using some algorithms, for example, Blast (Altschul et al. (1997) Nucleic Acids Res. 25:3389-3402), Blast2 (Altschul et al. (1990) J. Mol. Biol. 215:403-410), Smith-Waterman (Smith et al. (1981) J. Mol. Biol. 147:195-197), and ClustalW.

In the present application, the terms "S100A8/S100A9", "S100A8/A9", "S100A8/S100A9 dimer", "S100A8/S100A9 heterodimer", "S100A8/S100A9 polypeptide", "S100A8/S100A9 protein", "dimers of S100A8 and S100A9", "heterodimers of S100A8 and S100A9", and other similar terms have the same meaning and used interchangeably to refer to a protein complex in a heterodimeric conformation formed by calcium-binding protein A8 (S100A8) and calcium-binding protein A9 (S100A9) under the presence of calcium and zinc ions.

In the present application, the term "antibody" refers to a specific immunoglobulin to an antigenic site. Antibodies may be produced according to well-known methods in the art. Antibody forms include polyclonal or monoclonal antibodies, antibody fragments (e.g., Fab, Fab', F(ab')2, and Fv fragments), single-chain Fv (scFv) antibodies, multispecific antibodies (e.g., bispecific antibodies), monospecific antibodies, monovalent antibodies; chimeric antibodies, fully human antibodies, human antibodies, fusion proteins comprising the antigen-binding site of an antibody, and any other modified immunoglobulin molecules comprising an antigen-binding site, provided that such antibodies exhibit the desired biological binding activity.

In the present application, the term "multispecific" refers to that an antigen-binding protein has multiepitope specificity (i.e., capable of specifically binding to two, three, or more different epitopes on the same biomolecule, or epitopes on two, three, or more different biomolecules). In the present application, the term "bispecific" refers to that an antigen-binding protein has two distinct antigen-binding specificities.

The precise amino acid sequence boundaries of a given complementarity-determining region (CDR) or framework region (FR) can be readily determined using many well-known numbering schemes in the art. These schemes include: Kabat et al. (1991), "Sequences of Proteins of Immunological Interest", 5th ed., Public Health Service, National Institutes of Health, Bethesda, Maryland (the "Kabat" numbering scheme); Al-Lazikani et al., (1997) JMB 273, 927-948 (the "Chothia" numbering scheme); MacCallum et al., J. Mol. Biol. 262:732-745 (1996), "Antibody-antigen interactions: Contact analysis and binding site topography" J. Mol. Biol. 262, 732-745 (the "Contact" numbering scheme); Lefranc M.P. et al., "IMGT unique numbering for immunoglobulin and T cell receptor variable domains and Ig superfamily V-like domains", Dev Comp Immunol, January 2003; 27(1):55-77 (the "IMGT" numbering scheme); Honegger A and Plückthun A, "Yet another numbering scheme for immunoglobulin variable domains: an automatic modeling and analysis tool", J. Mol. Biol., June 8, 2001; 309(3):657-70 (the "Aho" numbering scheme); and Martin et al., "Modeling antibody hypervariable loops: a combined algorithm", PNAS, 1989, 86(23):9268-9272 (the "AbM" numbering scheme).

The boundaries of a given CDR or FR may vary depending on the scheme used for identification. For instance, the Kabat scheme is based on structural alignment, whereas the Chothia scheme is based on structural information. The numbering is based on the most common sequence length of antibody regions in both the Kabat and Chothia schemes, where insertions are provided by inserting letters (e.g., "30a") and deletions occur in some antibodies. The two schemes place certain insertions and deletions (indels) at different positions, resulting in distinct numbering. The Contact scheme is based on the analysis of complex crystal structures and is similar to the Chothia numbering scheme in many aspects. The AbM scheme serves as a compromise between Kabat and Chothia definitions and is based on the scheme used by Oxford Molecular's AbM antibody modeling software.

Therefore, unless otherwise specified, it shall be understood that the term "CDR" of a given antibody or a region thereof (e.g., its variable region) encompasses CDRs as defined by any of the aforementioned schemes or other known schemes. For instance, in the case where a specific CDR (e.g., CDR3) is designated to contain a given amino acid sequence, it shall be understood that such CDR may also have sequences corresponding to the CDR (e.g., CDR3) as defined by any of the aforementioned schemes or other known schemes. Similarly, unless otherwise specified, it shall be understood that the term "FR" of a given antibody or a region thereof (e.g., its variable region) encompasses FRs as defined by any of the aforementioned schemes or other known schemes. Unless specifically indicated, the Kabat numbering scheme is adopted for determining CDR and FR boundaries used herein.

In the present application, the term "humanized" antibody refers to an antibody in which all or substantially all CDR amino acid residues originate from non-human CDRs, and all or substantially all FR amino acid residues originate from human FRs. A "humanized form" of a non-human antibody refers to a variant of the non-human antibody that has undergone humanization to generally reduce immunogenicity in humans while retaining the specificity and affinity of the parental non-human antibody. In some embodiments, certain FR residues in the humanized antibody are substituted with corresponding residues from the non-human antibody (e.g., the antibody from which CDR residues are derived), for instance, to restore or improve antibody specificity or affinity. Humanized antibodies and preparation methods therefor are well-known in the art, see, e.g., Almagro and Fransson, Front. Biosci. 13:1619-1633 (2008). Human framework regions suitable for humanization include, but are not limited to: framework regions selected using the "best-fit" method; framework regions derived from the consensus sequence of a specific subgroup of human antibody of light or heavy chain variable regions; human mature (somatic mutant) framework regions or human germline framework regions; and framework regions obtained by screening FR libraries.

In the present application, the term "gene" is the entire nucleotide sequence required to produce a polypeptide chain or functional RNA. Therefore, gene expression includes transcription and steady accumulation of an encoded RNA (mRNA) or functional RNA derived from the gene, and also includes the translation of mRNA into polypeptides or proteins.

It needs to be noted that in the context of the present application, "upstream" refers to the 5' end of a gene or the N-terminus of a protein; "downstream" refers to the 3' end of a gene or the C-terminus of a protein; the direction from upstream to downstream represents the direction from 5' end to 3' end or the direction from N-terminus to C-terminus.

In the present application, the terms "exogenous" and "heterologous" are used interchangeably to refer to a source distinct from the native (original) organism, for example, derived from an organism of another species. In the present application, the term "heterologous gene" or "exogenous gene" refers to a gene not naturally present in a host organism, which is introduced into the host organism through gene transfer.

In the present application, the term "vector" refers to a self-replicating DNA molecule capable of transferring an exogenous target gene into a host organism, typically existing as a circular double-stranded DNA molecule. Representative vectors include plasmids, viruses, bacteriophages, cosmids, and microchromosomes. Among these, plasmids constitute the most common vector form and refer to circular double-stranded DNAs that can accept exogenous nucleic acid fragments and replicate within prokaryotic or eukaryotic cells.

In the present application, the terms "expression vector" and "recombinant vector" are used interchangeably to refer to a vector containing an exogenous gene, and also containing regulatory elements that enable expression in a specified host organism. Introduction of the expression vector into an appropriate host organism enables expression of the inserted target gene.

In the present application, the term "transformation" refers to the transfer of an exogenous gene into a host organism (e.g., a host cell), resulting in stable inheritance of the gene. The transformed gene may be maintained as a plasmid within the host organism or integrated into the genome of the host organism. A host organism containing the transformed gene is termed a "transgenic", "recombinant", "transformed", or "engineered" organism. Transformation of the expression vector into the host organism may be performed using routine techniques well-known to those skilled in the art. When the host is a procaryotic organism, competent cells capable of DNA uptake may be harvested after the logarithmic growth phase and treated via the CaCl₂ method, with employed procedures well-known in the art. Methods such as microinjection, electroporation, or liposome packing may be employed if required. These constitute well-known techniques in the art and thus require no detailed elaboration herein.

In the present application, the terms "amelioration" and "treatment" and their synonyms refer to an improvement in a disease, disorder, and/or condition. The terms "amelioration" and "treatment" may refer to an improvement in at least one measurable physical parameter, which is not necessarily discernible by the patient. The terms "amelioration" and "treatment" may also refer to inhibition of the progression of the disease, disorder, and/or condition physically (e.g., stabilizing recognizable symptoms), physiologically (e.g., stabilizing physical parameters), or both. The terms "amelioration" and "treatment" may also refer to slowing or reversal of the progression of the disease, disorder, and/or condition.

In the present application, the term "prevention" and its synonyms refer to delaying the onset of a specific disease, disorder, and/or condition, or symptoms associated therewith; or reducing the risk of developing the disease, disorder, and/or condition.

To render the foregoing objectives, features, and advantages of the present application more clear and comprehensible, the specific embodiments of the present application will be described in detail below.

### Antibody or Antigen-Binding Fragment

According to one embodiment of the present application, provided is a fully human antibody or an antigen-binding fragment thereof, wherein complementarity-determining regions (CDRs) thereof defined according to a KABAT system have the following amino acid sequences: an amino acid sequence of heavy chain CDR1 comprises GX₁TX₂SX₃X₄X₅X₆X₇, wherein X₁ is F or Y, X₂ is F, H, Y, or W, X₃ is S or D, X₄ is Y, H, or F, X₅ is A or V, X₆ is M, I, or L, and X₇ is S or A; an amino acid sequence of heavy chain CDR2 comprises AISGX₈GGSX₉X₁₀YX₁₁X₁₂SVKG, wherein X₈ is S or H, X₉ is T or H, X₁₀ is Y, W, or F, X₁₁ is A, H, V, or L, and X₁₂ is D, A, or H; an amino acid sequence of heavy chain CDR3 comprises KX₁₃RPX₁₄RX₁₅X₁₆DX₁₇, wherein X₁₃ is Y, F, or H, X₁₄ is S, T, or H, X₁₅ is V or A, X₁₆ is F, Y, H, or W, and X₁₇ is S, A, F, C, H, or Y; an amino acid sequence of light chain CDR1 comprises SGDALX₁₈DKX₁₉X₂₀X₂₁, wherein X₁₈ is G or H, X₁₉ is Y or F, X₂₀ is A or M, and X₂₁ is S or A; an amino acid sequence of light chain CDR2 comprises X₂₂X₂₃X₂₄X₂₅RX₂₆X₂₇, wherein X₂₂ is E or Q, X₂₃ is D, H, or Q, X₂₄ is S, A, or H, X₂₅ is K, H, R, P, or N, X₂₆ is P, Q, D, or N, and X₂₇ is S, D, Y, or W; and an amino acid sequence of light chain CDR3 comprises X₂₈SNDX₂₉DX₃₀X₃₁W, wherein X₂₈ is Q or D, X₂₉ is A, V, I, or L, X₃₀ is S or A, and X₃₁ is V, Y, W, or R.

In some embodiments, the CDRs of the antibody or antigen-binding fragment thereof have the following amino acid sequences: amino acid sequences of heavy chain CDRs comprise SEQ ID NOs: 1-3 and 23-64; and amino acid sequences of light chain CDRs comprise SEQ ID NOs: 4-6 and 65-109.

In some embodiments, the CDRs of the antibody or antigen-binding fragment thereof have the following amino acid sequences: the heavy chain CDR1 is selected from at least one of amino acid sequences shown in SEQ ID NOs: 1 and 23-34; the heavy chain CDR2 is selected from at least one of amino acid sequences shown in SEQ ID NOs: 2 and 35-48; the heavy chain CDR3 is selected from at least one of amino acid sequences shown in SEQ ID NOs: 3 and 49-64; the light chain CDR1 is selected from at least one of amino acid sequences shown in SEQ ID NOs: 4 and 65-69; the light chain CDR2 is selected from at least one of amino acid sequences shown in SEQ ID NOs: 5 and 70-95; and the light chain CDR3 is selected from at least one of amino acid sequences shown in SEQ ID NOs: 6 and 96-109.

In some embodiments, the amino acid sequences of the CDRs of the antibody or antigen-binding fragment thereof are selected from at least one of the following groups (1)-(119):
(1) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 6, respectively;
(2) the heavy chain CDR1, the heavy chain CDR2, the heavy chain CDR3, the light chain CDR1, the light chain CDR2, and the light chain CDR3 are amino acid sequences shown in SEQ ID NOs: 24, 2, 3, 4, 5, and 6, respectively;
(3) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 3, 4, 5, and 6, respectively;
(4) the heavy chain CDR1, the heavy chain CDR2, the heavy chain CDR3, the light chain CDR1, the light chain CDR2, and the light chain CDR3 are amino acid sequences shown in SEQ ID NOs: 26, 2, 3, 4, 5, and 6, respectively;
(5) the heavy chain CDR1, the heavy chain CDR2, the heavy chain CDR3, the light chain CDR1, the light chain CDR2, and the light chain CDR3 are amino acid sequences shown in SEQ ID NOs: 29, 2, 3, 4, 5, and 6, respectively;
(6) the heavy chain CDR1, the heavy chain CDR2, the heavy chain CDR3, the light chain CDR1, the light chain CDR2, and the light chain CDR3 are amino acid sequences shown in SEQ ID NOs: 30, 2, 3, 4, 5, and 6, respectively;
(7) the heavy chain CDR1, the heavy chain CDR2, the heavy chain CDR3, the light chain CDR1, the light chain CDR2, and the light chain CDR3 are amino acid sequences shown in SEQ ID NOs: 31, 2, 3, 4, 5, and 6, respectively;
(8) the heavy chain CDR1, the heavy chain CDR2, the heavy chain CDR3, the light chain CDR1, the light chain CDR2, and the light chain CDR3 are amino acid sequences shown in SEQ ID NOs: 32, 2, 3, 4, 5, and 6, respectively;
(9) the heavy chain CDR1, the heavy chain CDR2, the heavy chain CDR3, the light chain CDR1, the light chain CDR2, and the light chain CDR3 are amino acid sequences shown in SEQ ID NOs: 33, 2, 3, 4, 5, and 6, respectively;
(10) the heavy chain CDR1, the heavy chain CDR2, the heavy chain CDR3, the light chain CDR1, the light chain CDR2, and the light chain CDR3 are amino acid sequences shown in SEQ ID NOs: 1, 37, 3, 4, 5, and 6, respectively;
(11) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 3, 4, 5, and 6, respectively;
(12) the heavy chain CDR1, the heavy chain CDR2, the heavy chain CDR3, the light chain CDR1, the light chain CDR2, and the light chain CDR3 are amino acid sequences shown in SEQ ID NOs: 1, 39, 3, 4, 5, and 6, respectively;
(13) the heavy chain CDR1, the heavy chain CDR2, the heavy chain CDR3, the light chain CDR1, the light chain CDR2, and the light chain CDR3 are amino acid sequences shown in SEQ ID NOs: 1, 40, 3, 4, 5, and 6, respectively;
(14) the heavy chain CDR1, the heavy chain CDR2, the heavy chain CDR3, the light chain CDR1, the light chain CDR2, and the light chain CDR3 are amino acid sequences shown in SEQ ID NOs: 1, 41, 3, 4, 5, and 6, respectively;
(15) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 3, 4, 5, and 6, respectively;
(16) the heavy chain CDR1, the heavy chain CDR2, the heavy chain CDR3, the light chain CDR1, the light chain CDR2, and the light chain CDR3 are amino acid sequences shown in SEQ ID NOs: 1, 43, 3, 4, 5, and 6, respectively;
(17) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 49, 4, 5, and 6, respectively;
(18) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 50, 4, 5, and 6, respectively;
(19) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 51, 4, 5, and 6, respectively;
(20) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 52, 4, 5, and 6, respectively;
(21) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 53, 4, 5, and 6, respectively;
(22) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 54, 4, 5, and 6, respectively;
(23) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 55, 4, 5, and 6, respectively;
(24) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 56, 4, 5, and 6, respectively;
(25) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 5, and 6, respectively;
(26) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 66, 5, and 6, respectively;
(27) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 70, and 6, respectively;
(28) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 71, and 6, respectively;
(29) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 72, and 6, respectively;
(30) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 73, and 6, respectively;
(31) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 74, and 6, respectively;
(32) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 75, and 6, respectively;
(33) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 76, and 6, respectively;
(34) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 77, and 6, respectively;
(35) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 96, respectively;
(36) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 97, respectively;
(37) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 98, respectively;
(38) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 99, respectively;
(39) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 100, respectively;
(40) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 3, 4, 5, and 97, respectively;
(41) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 5, and 100, respectively;
(42) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 5, and 99, respectively;
(43) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 6, respectively;
(44) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 73, and 100, respectively;
(45) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 3, 4, 73, and 100, respectively;
(46) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 53, 66, 5, and 6, respectively;
(47) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 4, 5, and 99, respectively;
(48) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 66, 5, and 99, respectively;
(49) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 57, 4, 73, and 99, respectively;
(50) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 100, respectively;
(51) the heavy chain CDR1, the heavy chain CDR2, the heavy chain CDR3, the light chain CDR1, the light chain CDR2, and the light chain CDR3 are amino acid sequences shown in SEQ ID NOs: 1, 31, 57, 4, 76, and 100, respectively;
(52) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 4, 73, and 99, respectively;
(53) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 53, 4, 73, and 100, respectively;
(54) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 66, 5, and 99, respectively;
(55) the heavy chain CDR1, the heavy chain CDR2, the heavy chain CDR3, the light chain CDR1, the light chain CDR2, and the light chain CDR3 are amino acid sequences shown in SEQ ID NOs: 1, 39, 53, 66, 5, and 100, respectively;
(56) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 104, respectively;
(57) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 66, 73, and 104, respectively;
(58) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 57, 4, 73, and 104, respectively;
(59) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 64, 4, 73, and 104, respectively;
(60) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 4, 73, and 104, respectively;
(61) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 57, 4, 85, and 100, respectively;
(62) the heavy chain CDR1, the heavy chain CDR2, the heavy chain CDR3, the light chain CDR1, the light chain CDR2, and the light chain CDR3 are amino acid sequences shown in SEQ ID NOs: 31, 42, 57, 4, 87, and 100, respectively;
(63) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 57, 4, 73, and 106, respectively;
(64) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 66, 73, and 100, respectively;
(65) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 57, 4, 85, and 104, respectively;
(66) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 4, 73, and 104, respectively;
(67) the heavy chain CDR1, the heavy chain CDR2, the heavy chain CDR3, the light chain CDR1, the light chain CDR2, and the light chain CDR3 are amino acid sequences shown in SEQ ID NOs: 29, 42, 62, 4, 87, and 100, respectively;
(68) the heavy chain CDR1, the heavy chain CDR2, the heavy chain CDR3, the light chain CDR1, the light chain CDR2, and the light chain CDR3 are amino acid sequences shown in SEQ ID NOs: 32, 42, 63, 4, 73, and 105, respectively;
(69) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 63, 4, 85, and 100, respectively;
(70) the heavy chain CDR1, the heavy chain CDR2, the heavy chain CDR3, the light chain CDR1, the light chain CDR2, and the light chain CDR3 are amino acid sequences shown in SEQ ID NOs: 1, 47, 63, 66, 73, and 100, respectively;
(71) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 66, 73, and 104, respectively;
(72) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 66, 73, and 104, respectively;
(73) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 4, 85, and 104, respectively;
(74) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 64, 4, 87, and 104, respectively;
(75) the heavy chain CDR1, the heavy chain CDR2, the heavy chain CDR3, the light chain CDR1, the light chain CDR2, and the light chain CDR3 are amino acid sequences shown in SEQ ID NOs: 1, 48, 63, 66, 85, and 100, respectively;
(76) the heavy chain CDR1, the heavy chain CDR2, the heavy chain CDR3, the light chain CDR1, the light chain CDR2, and the light chain CDR3 are amino acid sequences shown in SEQ ID NOs: 1, 43, 63, 66, 73, and 106, respectively;
(77) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 88, and 100, respectively;
(78) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 89, and 100, respectively;
(79) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 90, and 100, respectively;
(80) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 92, and 100, respectively;
(81) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 91, and 100, respectively;
(82) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 94, and 100, respectively;
(83) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 93, and 100, respectively;
(84) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 93, and 109, respectively;
(85) the heavy chain CDR1, the heavy chain CDR2, the heavy chain CDR3, the light chain CDR1, the light chain CDR2, and the light chain CDR3 are amino acid sequences shown in SEQ ID NOs: 23, 42, 57, 4, 73, and 100, respectively;
(86) the heavy chain CDR1, the heavy chain CDR2, the heavy chain CDR3, the light chain CDR1, the light chain CDR2, and the light chain CDR3 are amino acid sequences shown in SEQ ID NOs: 29, 42, 57, 4, 73, and 100, respectively;
(87) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 108, respectively;
(88) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 107, respectively;
(89) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 109, respectively;
(90) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 73, and 100, respectively;
(91) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 69, 73, and 100, respectively;
(92) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 73, and 109, respectively;
(93) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 93, and 109, respectively;
(94) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 86, and 100, respectively;
(95) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 86, and 100, respectively;
(96) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 67, 73, and 108, respectively;
(97) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 95, and 108, respectively;
(98) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 94, and 108, respectively;
(99) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 100, respectively;
(100) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 73, and 100, respectively;
(101) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 68, 73, and 100, respectively;
(102) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 108, respectively;
(103) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 107, respectively;
(104) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 109, respectively;
(105) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 69, 73, and 100, respectively;
(106) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 73, and 109, respectively;
(107) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 86, and 100, respectively;
(108) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 93, and 109, respectively;
(109) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 86, and 100, respectively;
(110) the heavy chain CDR1, the heavy chain CDR2, the heavy chain CDR3, the light chain CDR1, the light chain CDR2, and the light chain CDR3 are amino acid sequences shown in SEQ ID NOs: 34, 42, 57, 65, 73, and 100, respectively;
(111) the heavy chain CDR1, the heavy chain CDR2, the heavy chain CDR3, the light chain CDR1, the light chain CDR2, and the light chain CDR3 are amino acid sequences shown in SEQ ID NOs: 1, 44, 57, 68, 73, and 100, respectively;
(112) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 65, 73, and 100, respectively;
(113) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 69, 73, and 100, respectively;
(114) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 4, 86, and 100, respectively;
(115) the heavy chain CDR1, the heavy chain CDR2, the heavy chain CDR3, the light chain CDR1, the light chain CDR2, and the light chain CDR3 are amino acid sequences shown in SEQ ID NOs: 28, 42, 57, 4, 73, and 108, respectively;
(116) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 59, 4, 73, and 108, respectively;
(117) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 58, 4, 73, and 108, respectively;
(118) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 60, 4, 73, and 108, respectively; and
(119) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 61, 4, 73, and 108, respectively.

In some embodiments, the antibody or antigen-binding fragment thereof further comprises framework regions (FRs). Suitable FR sequences are known in the art, and any suitable FR sequences may be used in the present application. In some embodiments, the heavy chain FRs comprise FRs within the heavy chain variable domain shown in SEQ ID NO: 7. In some embodiments, the light chain FRs comprise FRs within the light chain variable domain shown in SEQ ID NO: 8.

In some embodiments, a heavy chain variable region of the antibody or antigen-binding fragment thereof has an amino acid sequence having at least 75%, 80%, 85%, 90%, 95%, 98%, 99%, or 100% identity to a sequence shown in SEQ ID NO: 7, and a light chain variable region thereof has an amino acid sequence having at least 75%, 80%, 85%, 90%, 95%, 98%, 99%, or 100% identity to a sequence shown in SEQ ID NO: 8. In some preferred embodiments, the heavy chain variable region of the antibody or antigen-binding fragment thereof comprises a sequence shown in SEQ ID NO: 7, and the light chain variable region thereof comprises a sequence shown in SEQ ID NO: 8.

In some embodiments, a heavy chain of the antibody or antigen-binding fragment thereof is an amino acid sequence obtained by mutation of at least 1, 2, 3, 4, 5, 6, 7, 8, 9, 10, 11, 12, 13, 14, 15, 16, or 17 positions occurring on a sequence shown in SEQ ID NO: 11. In some embodiments, the mutation comprises an insertion, deletion, and/or substitution. In some preferred embodiments, the mutation is a substitution. In some embodiments, an amino acid position for the substitution is selected from at least one of positions 27, 29, 31, 32, 33, 34, 35, 54, 58, 59, 61, 62, 100, 103, 105, 106, and 108 of the sequence shown in SEQ ID NO: 11. In some preferred embodiments, the amino acid position for the substitution is selected from at least one of F27Y, F29H, F29Y, F29W, S31D, Y32F, Y32H, A33V, M34I, M34L, S35A, S54H, T58H, Y59F, Y59W, A61H, A61V, A61L, D62A, D62H, Y100H, Y100F, S103H, S103T, V105A, F106H, F106Y, F106W, S108A, S108C, S108F, S108H, and S108Y.

In some embodiments, a light chain of the antibody or antigen-binding fragment thereof is an amino acid sequence obtained by mutation of at least 1, 2, 3, 4, 5, 6, 7, 8, 9, 10, 11, 12, 13, or 14 positions occurring on a sequence shown in SEQ ID NO: 12. In some embodiments, the mutation comprises an insertion, deletion, and/or substitution. In some preferred embodiments, the mutation is a substitution. In some embodiments, an amino acid position for the substitution is selected from at least one of positions 28, 31, 32, 33, 49, 50, 51, 52, 54, 55, 88, 92, 94, and 95 of the sequence shown in SEQ ID NO: 12. In some preferred embodiments, the amino acid position for the substitution is selected from at least one of G28H, Y31F, A32M, S33A, E49Q, D50H, D50Q, S51A, S51H, K52H, K52R, K52P, K52N, P54D, P54N, P54Q, S55D, S55W, S55Y, Q88D, A92V, A92L, A92I, S94A, V95R, V95W, and V95Y.

In some embodiments, the antibody or antigen-binding fragment thereof further comprises constant regions. Suitable constant region sequences are known in the art, and any suitable constant region sequences may be used in the present application. In some preferred embodiments, an amino acid sequence of a heavy chain constant region is shown in SEQ ID NO: 9, and an amino acid sequence of a light chain constant region is shown in SEQ ID NO: 10.

In some embodiments, the antibody or antigen-binding fragment thereof optionally further comprises signal peptides. When the antibody or antigen-binding fragment thereof is expressed and purified using a prokaryotic expression system, suitable signal peptide sequences are known in the art, and any suitable signal peptide sequences may be used in the present application. In some embodiments, the heavy chain comprises a heavy chain signal peptide shown in SEQ ID NO: 13, and the light chain comprises a light chain signal peptide shown in SEQ ID NO: 14. In some preferred embodiments, the heavy chain comprising the signal peptide has a sequence shown in SEQ ID NO: 15, and the light chain comprising the signal peptide has a sequence shown in SEQ ID NO: 16.

In some embodiments, the antibody or antigen-binding fragment thereof does not comprise signal peptides. When the antibody or antigen-binding fragment thereof is expressed and purified using a prokaryotic expression system, the heavy chain and the light chain may not comprise signal peptides. In some preferred embodiments, the heavy chain not comprising the signal peptide has a sequence shown in SEQ ID NO: 11, and the light chain not comprising the signal peptide has a sequence shown in SEQ ID NO: 12.

In some embodiments, the antibody or antigen-binding fragment thereof includes, but is not limited to: a single-chain antibody, a single-domain antibody, a diabody, a triabody, a disulfide-stabilized antibody, a nanobody, a Fab fragment, a Fab' fragment, a (Fab')2 fragment, a minibody, a monoclonal antibody, a polyclonal antibody, a monospecific antibody, a bispecific antibody, a multispecific antibody, a monovalent antibody, a multivalent antibody, a chimeric antibody, a fusion protein comprising an antigen-binding site of an antibody, or a full-length antibody immunoglobulin IgG1, IgG2, IgG3, IgG4, IgA, IgE, IgM, or IgD.

### Polynucleotide, Recombinant Vector, and Host Cell

According to one embodiment of the present application, further provided is a polynucleotide, encoding the antibody or antigen-binding fragment thereof of the present application.

According to one embodiment of the present application, further provided is a recombinant vector, comprising the polynucleotide of the present application. Recombinant vectors suitable for expressing the antibody or antigen-binding fragment thereof of the present application are well-known in the art and are not limited herein.

In some embodiments, the recombinant vector further comprises a promoter. The promoter may be any suitable promoter sequence, i.e., a nucleic acid sequence recognizable by host cells for expressing the nucleic acid sequence. The promoter sequence comprises transcriptional regulatory sequences that mediate expression of the antibody or antigen-binding fragment thereof. The promoter may be any nucleic acid sequence exhibiting transcriptional activity in selected host cells, including mutated, truncated, and hybrid promoters, and may be derived from genes encoding extracellular or intracellular proteins or polypeptides homologous or heterologous to the host cells.

The recombinant vector of the present application may be constructed using methods well-known in the art. For example, suitable restriction sites are added at both ends of the polynucleotide of the present application according to the restriction sites contained in the backbone vector used, followed by ligation into the backbone vector.

According to one embodiment of the present application, further provided is a host cell, comprising the polynucleotide of the present application and/or the recombinant vector of the present application. The host cell may be selected according to the type of expression vector. Any suitable method well-known in the art may be employed to deliver the polynucleotide and/or the expression vector into the host cell, with no limitation imposed herein.

### Pharmaceutical Composition and Kit

According to one embodiment of the present application, further provided is a pharmaceutical composition, comprising the antibody or antigen-binding fragment thereof of the present application.

In some embodiments, the pharmaceutical composition further comprises a pharmaceutically or physiologically acceptable carrier. The carrier may be any compatible, physiologically acceptable, non-toxic substance suitable for delivering the polypeptide, polynucleotide, or recombinant vector provided by the present application into a mammal (e.g., a human).

The term "pharmaceutically acceptable carrier" refers to a carrier, diluent, or adjuvant used in the formulation or administration of the polypeptide, polynucleotide, or recombinant vector provided by the present application. Such a carrier is not per se an essential active ingredient and exhibits no excessive toxicity after administration. Suitable pharmaceutically acceptable carriers are well-known to those of ordinary skill in the art.

The term "physiologically acceptable carrier" refers to a carrier, diluent, or adjuvant that does not induce significant irritation in a biological organism and does not abrogate the pharmaceutical activity or properties of the administered polypeptide, polynucleotide, or recombinant vector provided by the present application. Suitable physiologically acceptable carriers are also well-known to those of ordinary skill in the art.

In some embodiments, the pharmaceutical composition further comprises a pharmaceutically acceptable excipient. In some embodiments, the excipient comprises at least one of a solubilizer, a disintegrant, a wetting agent, a stabilizer, a thickener, a diluent, a buffer, and a flavoring agent.

In some non-limiting embodiments, carriers and/or excipients for the pharmaceutical composition in the present application may comprise, for example: liquids, gel or solid carriers, aqueous vehicles, non-aqueous vehicles, antimicrobial agents, isotonic agents, buffering agents, antioxidants, suspending agents, dispersing agents, chelating agents, diluents, adjuvants, excipients or nontoxic auxiliary substances, and other components known in the art or various combinations thereof.

According to one embodiment of the present application, further provided is a kit, comprising the antibody or antigen-binding fragment thereof of the present application.

### Multispecific Antibody and Antibody Conjugate

According to one embodiment of the present application, further provided is a multispecific antibody, comprising the antibody or antigen-binding fragment thereof of the present application, and further comprising one or more second antibodies or antigen-binding fragments thereof which bind specifically to other antigens.

In some embodiments, the second antibody or the antigen-binding fragment thereof is selected from a full-length antibody, a Fab, a Fab", a (Fab")2, an Fv, an scFv, an scFv-scFv, a minibody, a diabody, or an sdAb.

Those skilled in the art may select a suitable second antibody or antigen-binding fragment thereof as needed, and conjugate the antibody or antigen-binding fragment provided in the present application with the second antibody or antigen-binding fragment thereof using methods known in the art to form a multispecific antibody.

According to one embodiment of the present application, further provided is an antibody conjugate, comprising the antibody or antigen-binding fragment thereof of the present application, and a second functional structure selected from an Fc, a radioisotope, a half-life extending structural moiety, a detectable label, and a drug.

Suitable Fc, radioisotopes, half-life extending structural moieties, detectable labels, drugs, and the like are known in the art. Those skilled in the art may select a suitable second functional structure as needed and conjugate the antibody or antigen-binding fragment provided in the present application with the second functional structure using methods known in the art.

In some embodiments, the antibody conjugate comprises the anti-S100A8/A9 antibody provided by the present application and an Fc. The term "Fc" is used to define the C-terminal region of an immunoglobulin heavy chain including natural Fc and variant Fc. The term "natural Fc" refers to a molecule or sequence comprising a non-antigen-binding fragment produced by digesting an intact antibody, whether in monomeric or polymeric form. The immunoglobulin source producing natural Fc is preferably of human origin. Natural Fc fragments consist of monomeric polypeptides that may link into dimeric or polymeric forms through covalent linkages (e.g., disulfide bonds) and noncovalent linkages. Depending on the class (e.g., IgG, IgA, IgE, IgD, IgM) or subclass (e.g., IgG1, IgG2, IgG3, IgA1, IgGA2), 1-4 intermolecular disulfide bonds exist between natural Fc molecule monomeric subunits. An example of natural Fc is the disulfide-linked dimer produced by papain digestion of IgG (see Ellison et al. (1982), Nucleic Acids Res. 10:4071-9). The term "natural Fc" used herein generally refers to monomeric, dimeric, and polymeric forms. The term "variant Fc" refers to an amino acid sequence differing from the amino acid sequence of "natural" or "wild-type" Fc due to at least one "amino acid modification" as defined herein, also termed "Fc variant". Thus, "Fc" also comprises single-chain Fc (scFc), that is, single-chain Fc formed by two Fc monomers connected by a polypeptide linker can naturally fold into a functional dimeric Fc region. In one embodiment, the Fc is preferably derived from human immunoglobulin, and more preferably from human IgG1.

In some embodiments, the antibody conjugate comprises the anti-S100A8/A9 antibody provided by the present application and a drug to form an antibody-drug conjugate (ADC), wherein the drug is selected from a cytotoxic agent and an immunomodulator. In some embodiments, examples of the cytotoxic agent include, but are not limited to: methotrexate, aminopterin, 6-mercaptopurine, 6-thioguanine, cytarabine, 5-fluorouracil, dacarbazine, mechlorethamine, thiotepa, chlorambucil, melphalan, carmustine (BSNU), lomustine (CCNU), 1-methylnitrosourea, cyclophosphamide, nitrogen mustard, busulfan, dibromomannitol, streptozocin, mitomycin, cis-dichlorodiammineplatinum(II) (DDP), cisplatin, carboplatin, zorubicin, doxorubicin, detorubicin, caminomycin, idarubicin, epirubicin, mitoxantrone, actinomycin D, bleomycin, calicheamicin, mithramycin, anthramycin (AMC), vincristine, vinblastine, paclitaxel, ricin, Pseudomonas exotoxin, gemcitabine, cytochalasin B, gramicidin D, ethidium bromide, emetine, etoposide, teniposide, colchicine, dantron, 1-dehydrotestosterone, glucocorticoid, procaine, tetracaine, lidocaine, propranolol, puromycin, procarbazine, hydroxyurea, asparaginase, corticosteroid, mitotane (O,P"-(DDD)), interferon, and combinations thereof. In some embodiments, examples of the immunomodulator include, but are not limited to: ganciclovir, etanercept, tacrolimus, sirolimus, voclosporin, cyclosporine, rapamycin, cyclophosphamide, azathioprine, mycophenolate mofetil, methotrexate, glucocorticoid and analogs thereof, cytokine, stem cell growth factor, lymphotoxin, tumor necrosis factor (TNF), hematopoietic factor, interleukins (e.g., IL-1, IL-2, IL-3, IL-6, IL-10, IL-12, IL-18, and IL-21), colony-stimulating factors (e.g., G-CSF and GM-CSF), interferons (e.g., interferon-α, interferon-β, and interferon-γ), erythropoietin, thrombopoietin, and combinations thereof.

In some embodiments, the antibody conjugate comprises the anti-S100A8/A9 antibody provided by the present application and a radioisotope to form a radionuclide drug conjugate (RDC). Examples of the radioisotope suitable for use in the present application include, but are not limited to: At211, I131, I125, Y90, Re186, Re188, Sm153, Bi212, P32, Pb212, 99mTc, 123I, 18F, and 68Ga.

In some embodiments, the antibody conjugate comprises the anti-S100A8/A9 antibody provided by the present application and a half-life extending structural moiety to improve the half-life of the anti-S100A8/A9 antibody. The half-life extending structural moiety is selected from an albumin-binding structure, a transferrin-binding structure, a polyethylene glycol molecule, a recombinant polyethylene glycol molecule, a human serum albumin, a human serum albumin fragment, and a human serum albumin-binding polypeptide.

In some embodiments, the antibody conjugate comprises the anti-S100A8/A9 antibody provided by the present application and a detectable label. The detectable label is selected from a fluorophore, a chemiluminescent compound, a bioluminescent compound, an enzyme, an antibiotic resistance gene, and a contrast agent. The term "detectable label" in the present application refers to a compound that generates a detectable signal. For example, the detectable label may be an MRI contrast agent, a scintigraphic contrast agent, an X-ray imaging contrast agent, an ultrasound contrast agent, or an optical imaging contrast agent. Examples of the detectable label include fluorophores (e.g., fluorescein, Alexa, or cyanine), chemiluminescent compounds (e.g., luminol), bioluminescent compounds (e.g., luciferase or alkaline phosphatase), enzymes (e.g., horseradish peroxidase, glucose-6-phosphatase, β-galactosidase), antibiotic (e.g., kanamycin, ampicillin, chloramphenicol, tetracycline, etc.) resistance genes, and contrast agents (e.g., nanoparticles or gadolinium). Those skilled in the art may select a suitable detectable label according to the detection system employed.

### Use

According to one embodiment of the present application, further provided is use of the antibody or antigen-binding fragment thereof of the present application, the multispecific antibody of the present application, and/or the antibody conjugate of the present application in preparing a drug, a reagent, or a kit for detecting, preventing, or treating a disease.

In some embodiments, the disease is associated with elevated expression levels of oligomers, dimers, tetramers, or higher-order multimers of S100A8 and S100A9. Within the scope of what is known in the art, various diseases or non-disease conditions are associated with elevated expression levels of oligomers, dimers, tetramers, or higher-order multimers of S100A8/S100A9. The diseases or non-disease conditions include, but are not limited to: sepsis (Am J Respir Crit Care Med, 2017, 196(3):315-327), autoimmune diseases (Autoimmun Rev, 2023, 22(5):103295), acute lung injury (Blood, 2022, 140(24):2626-2643), acute pancreatitis (Dis Markers, 2018, 2018:6457347), acute kidney injury (Adv Sci (Weinh), 2022, 9(12):e2103675), tumor (Sci Transl Med, 2020, 12(572):eabb5817), organ ischemia/reperfusion injury *(*Circulation, 2019, 140(9):751-764; Kidney Int, 2015, 87(1):85-94), and atherosclerosis-related cardiovascular and cerebrovascular diseases (Pharmacol Res, 2020, 161:105212). The disclosures are incorporated herein by reference in their entirety. In some preferred embodiments, the diseases comprise myocardial infarction and myocardial ischemia/reperfusion injury.

According to one embodiment of the present application, further provided is use of the antibody or antigen-binding fragment thereof of the present application, the multispecific antibody of the present application, and/or the antibody conjugate of the present application in preparing a drug, a reagent, or a kit for reducing a myocardial infarct size, improving a cardiac function, or improving a microcirculatory function.

In the use of the present application, the administration dose of the antibody or antigen-binding fragment provided by the present application may depend on several factors, including the severity and responsiveness of the symptom, the route of administration, the therapeutic duration (ranging from days to months to years), and the time required to achieve symptomatic improvement. Those skilled in the art may adjust the dosage regimen according to the specific patient circumstances to provide a therapeutic response. For instance, a single dose may be administered, several divided doses may be administered over a predetermined time period, or the dose may be reduced or increased as the therapeutic situation dictates. The specification of the dose is determined by the specific therapeutic effect to be achieved. Dose values may also vary with the type and severity of the condition to be alleviated. For any particular subject, the specific dosage regimen may be adjusted over time according to individual needs and the professional judgment of the therapeutic clinician.

### Method for Detection, Prevention, or Treatment of Diseases

According to one embodiment of the present application, further provided is a method for detecting, preventing, or treating a disease, comprising administrating to a subject in need thereof the antibody or antigen-binding fragment thereof of the present application, the pharmaceutical composition of the present application, the kit of the present application, the multispecific antibody of the present application, and/or the antibody conjugate of the present application.

In some embodiments, the disease is associated with elevated expression levels of oligomers, dimers, tetramers, or higher-order multimers of S100A8 and S100A9. Within the scope of what is known in the art, various diseases or non-disease conditions are associated with elevated expression levels of oligomers, dimers, tetramers, or higher-order multimers of S100A8/S100A9. The diseases or non-disease conditions include, but are not limited to: Sepsis (Am J Respir Crit Care Med, 2017, 196(3):315-327), Autoimmune diseases (Autoimmun Rev, 2023, 22(5):103295), Acute lung injury (Blood, 2022, 140(24):2626-2643), Acute pancreatitis (Dis Markers, 2018, 2018:6457347), *Acute kidney injury* (Adv Sci (Weinh), 2022, 9(12):e2103675), Cancer (Sci Transl Med, 2020, 12(572):eabb5817), Organ ischemia/reperfusion injury *(*Circulation, 2019, 140(9):751-764; Kidney Int, 2015, 87(1):85-94), and Atherosclerosis-related cardiovascular and cerebrovascular diseases (Pharmacol Res, 2020, 161:105212). The disclosures are incorporated herein by reference in their entirety. In some preferred embodiments, the disease comprises ischemic organ injury and organ ischemia/reperfusion injury related to S100A8/A9, S100A8, or S100A9.

According to one embodiment of the present application, further provided is a method for reducing a myocardial infarct size, improving a cardiac function, or improving a microcirculatory function, comprising administrating to a subject in need thereof the antibody or antigen-binding fragment thereof of the present application, the pharmaceutical composition of the present application, the kit of the present application, the multispecific antibody of the present application, and/or the antibody conjugate of the present application.

In the method of the present application, the administration dose of the antibody or antigen-binding fragment provided by the present application may depend on several factors, including the severity and responsiveness of the symptom, the route of administration, the therapeutic duration (ranging from days to months to years), and the time required to achieve symptomatic improvement. Those skilled in the art may adjust the dosage regimen according to the specific patient circumstances to provide a therapeutic response. For instance, a single dose may be administered, several divided doses may be administered over a predetermined time period, or the dose may be reduced or increased as the therapeutic situation dictates. The specification of the dose is determined by the specific therapeutic effect to be achieved. Dose values may also vary with the type and severity of the condition to be alleviated. For any particular subject, the specific dosage regimen may be adjusted over time according to individual needs and the professional judgment of the therapeutic clinician.

The various embodiments and preferred options disclosed above may be combined with each other (provided that they are not inherently contradictory). Any embodiments formed by such a combination are considered part of the disclosure of the present application.

The following describes exemplary embodiments of the present application with reference to the accompanying drawings. The description includes various details of the embodiments of the present application to aid understanding. It should be understood that they are considered merely exemplary and are in no way intended to limit the scope of protection of the present application. The scope of protection of the present application is defined solely by the claims. Therefore, those of ordinary skill in the art should recognize that various changes and modifications may be made to the embodiments described herein without departing from the scope of the present application. Similarly, descriptions of well-known functions and structures are omitted in the following description for clarity and conciseness.

### Examples

In the examples, specific techniques or conditions not specified shall be carried out according to the techniques or conditions described in the literature in the art or according to the product manual. Reagents or instruments for which the manufacturer is not specified are all commercially available conventional products.

### Example 1: Preparation of Prokaryotic Recombinant Antigens from Different Species

Gene fragments of S100A8 (Sino Biological, Cat. No. HG11138-CH, Uniprot: P05109; amino acid sequence shown as SEQ ID No: 110, coding nucleotide sequence shown as SEQ ID No: 111) and S100A9 (Sino Biological, Cat. No. HG11145-CH, Uniprot: P06702; amino acid sequence shown as SEQ ID No: 112, coding nucleotide sequence shown as SEQ ID No: 113) were purchased for protein expression in *Escherichia coli.* The gene fragments were cloned into the pET21a vector (Novagen) enzymatically cleaved by NdeI and BamHI, to construct pET21-S100A8 or pET21-S100A9 vectors. For the co-expression experiment, the pET21-S100A8-S100A9 co-expression vector was used. Recombinant proteins from different species, including human S100A8/A9 (hS100A8/A9), mouse S100A8/A9 (mS100A8/A9), and monkey S100A8/A9 (cS100A8/A9), were constructed and prepared using similar methods. A His-tag was designed at the C-terminus of the recombinant proteins for purification. The plasmids to be expressed were transformed into *Escherichia coli* BL21(DE3) T7 expression cells. Colonies were inoculated into 20 mL of LB medium containing 100 µg/mL ampicillin and cultured in a shaking flask for 2 hours at 37°C. The 20 mL pre-culture was inoculated into 800 mL of LB medium containing 100 µg/mL ampicillin. When the cell density reached an OD600 of 0.5, 0.5 mM isopropyl β-d-1-thiogalactopyranoside (IPTG) was added for induction for 3 hours. The *Escherichia coli* cells were harvested by centrifugation, and the centrifuged cells were washed with 0.15 M NaCl. The cells were resuspended in 80 mL of 50 mM Tris-HCl buffer (pH 7.5) containing 50 mM NaCl and 5 mM MgSO₄, followed by sonication on ice for cell lysis. The supernatant collected by centrifugation after sonication was purified using metal chelate affinity chromatography (Ni-NTA) to obtain the His-tagged fusion recombinant protein. The target protein collected after purification was dialyzed to exchange the buffer into PBS, dispensed and stored at -80°C. Depending on the requirements of different experiments, pyrogen removal was performed on the recombinant protein. The pyrogen-free recombinant protein was then dispensed and stored at -80°C.

### Example 2: Preparation and Activity Assay of Anti-S100A8/A9 Antibody

### 2.1 Screening the Phage Antibody Library for Anti-hS100A8/A9 Antibodies

Immunotubes were coated with the hS100A8/A9-His (hereinafter abbreviated as hS100A8/A9) antigen prepared in Example 1, with 20 µg/mL and 1 mL per tube, and incubated overnight at 4°C. The next day, the tubes were washed four times with PBS, with 3 minutes per wash, followed by blocking with 1.5 mL of 2% BSA for 2 hours at 37°C. A phage library (constructed in-house) presenting single-chain antibodies was blocked with a blocking buffer (2% BSA, 0.1% Tween-20) for 30 minutes at 37°C and then added to the immunotubes, with an input of 10⁹-10¹² per tube, for binding for 1 hour at 37°C. After thorough washing with PBST and PBS, 1 mL of 0.2 mol/L glycine-hydrochloric acid (pH 2.2) was added for elution, and shaking was carried out at room temperature for 10 minutes. The eluate was immediately neutralized to approximately pH 7.0 using 1 mol/L Tris (pH 8.8). The neutralized phage solution was used to infect activated XL1-Blue *Escherichia coli* cells in the logarithmic growth phase. The mixture was standing in a 37°C incubator for 20-30 minutes and then placed in a shaker for shaking culture for 30 minutes at 37°C and 150 rpm. Subsequently, 1% and 0.1% of the bacterial solution were plated separately for counting. The remaining bacterial solution was centrifuged at 4,000 rpm for 10 minutes. The supernatant was discarded. The bacterial cells were spread onto 2YTCG solid plates, followed by overnight culture at 37°C. The next day, the bacterial cells from the plates were collected. An appropriate amount of the bacterial cells was inoculated into 2YTCG liquid medium and cultured with shaking until the logarithmic growth phase was reached. The bacterial cells were then infected with M13KO7 and standing at room temperature for 15-30 minutes, followed by culture for 1 hour at 37°C and 150 rpm. 50 µg/mL Kanamycin was then added. Overnight culture was carried out at 30°C. The next day, the phages were precipitated and purified using PEG8000/NaCl. The purified phages were then ready for the next round of panning. Three rounds of phage library enrichment screening were performed in total.

### 2.2 Monoclonal Identification of Anti-hS100A8/A9 Single-Chain Antibodies

Single colonies obtained after three rounds of phage screening were picked, inoculated into 200 µL of 2YTCTG medium, and cultured at 37°C and 220 rpm until the logarithmic growth phase was reached. Subsequently, 10⁸ helper phages M13KO7 were added to each well. The mixtures were standing at room temperature for infection for 20 minutes, followed by culture for 1 hour at 37°C and 150 rpm. Subsequently, an equal volume of 2×YTCTKI medium (containing 50 µg/mL kanamycin and 0.2 mmol/L IPTG) was added, followed by overnight culture at 30°C and 200 rpm. The next day, the supernatants were collected by centrifugation. BSA was added to a final concentration of 2%. Tween-20 was added to a final concentration of 0.1%. The mixtures were standing for 15 minutes at 37°C for ELISA identification. A 96-well ELISA plate was coated with the hS100A8/A9 antigen, with 200 ng/well, and cultured overnight at 4°C. The next day, the ELISA plate was blocked with PBST-4% milk, at 250 µL/well, for 2 hours at 37°C. The blocking buffer was discarded. Blocked phage antibodies were added. The plate was standing for 1 hour at 37°C. The liquids were discarded. The plate was washed three times with PBST. Anti-M13 Antibody (HRP) (Sino Biological, Cat. No. 11973-MM05T-H) diluted in the blocking buffer was added to the ELISA plate. The plate was standing for 30 minutes at 37°C. The plate was washed three times with PBST. The OPD substrate solution was added. The plate was standing at room temperature for color development for approximately 10 minutes. The color development was stopped by adding 2M H₂SO₄. Optical density (OD) values were measured using a microplate reader.

### 2.3 Conversion of Single-Chain Antibodies to Full-Length Antibodies and Expression and Purification

Full-length antibodies were prepared from the anti-hS100A8/A9 single-chain antibodies obtained after screening. VL variable region genes were amplified using primers LF (the corresponding nucleotide sequence is shown in SEQ ID No: 19) and LR (the corresponding nucleotide sequence is shown in SEQ ID No: 20). VH variable region genes were amplified using primers HF (the corresponding nucleotide sequence is shown in SEQ ID No: 21) and HR (the corresponding nucleotide sequence is shown in SEQ ID No: 22). Vector pABL was used for cloning the VL variable region genes, and Vector pABG4 was used for cloning the VH variable region genes. VL and VH genes were separately cloned into the vectors via homologous recombination. After transformation into *Escherichia coli* Top10, monoclonal colonies were picked for sequencing identification of recombinant plasmids. Correct expression vectors for the heavy chain and light chain of fully human antibodies were selected. After plasmid extraction, the heavy chain and light chain plasmids were transfected into 293-T cells at a 1:1 molar ratio. Transient expression of full-length antibodies was carried out. Expression supernatants were purified by ProteinA affinity chromatography. Purified full-length antibodies were subjected to electrophoresis identification, and dispensed and stored for subsequent experimental identification. Pyrogen removal was carried out on the antibodies according to different experimental requirements.

### 2.4 Binding Activity Assay of Anti-S100A8/A9 Monoclonal Antibodies

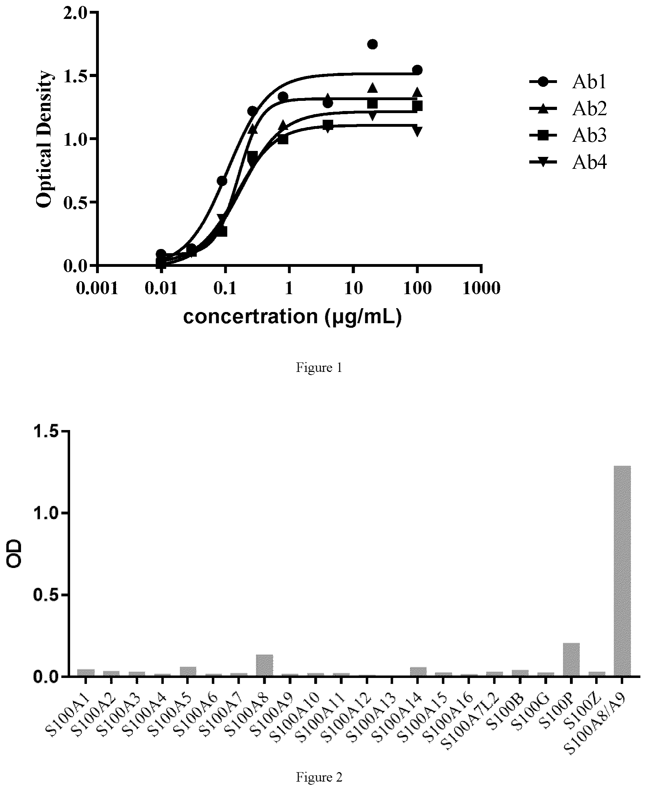

The binding activity of four candidate monoclonal antibodies to the hS100A8/A9 antigen was analyzed by ELISA. 96-well plates were coated with monoclonal antibodies (0.5 µg/mL, 50 µL/well, overnight at 4°C) obtained by expression and purification. The binding affinity of the monoclonal antibodies for the hS100A8/A9 antigen at different dilutions was determined. The starting concentration of the hS100A8/A9 antigen was 100 µg/mL, and 5-fold or 3-fold gradient dilution was carried out, with 8 dilutions set. HRP-conjugated 6×His, His-Tag Monoclonal antibody (Proteintech, Cat. No. HRP-66005) was used as an HRP-conjugated secondary antibody (results shown in Figure 1), with sequence-related information shown in Table 1 below.

**Table 1 Antibody-Related Sequences**

| Name | Type | Sequence |
|---|---|---|
| Heavy Chain | Amino Acid | EVQLVESGGGLVQPGGSLRLSCAASGFTFSSYAMSWVRQAPGKGLEWVSAISGSGGS TYYADSVKGRFTISRDNSKNTLYLQMNSLRAEDTAVYYCARKYRPSRVFDSWGQGTL VTVSSASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCPPCPAPEF LGGPSVFLFPPKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKGQPREPQVYT LPPSQEEMTKNQVSLTCLVKGFYPSDIAVEWESNGQPENNYKTTPPVLDSDGSFFLYSR LTVDKSRWQEGNVFSCSVMHEALHNHYTQKSLSLSLG (SEQ ID NO: 11) |
| | Coding Nucleotide | GAAGTGCAATTGGTGGAAAGCGGTGGCGGTCTGGTGCAGCCGGGTGGCAGCCTGC GTCTGAGCTGCGCAGCGAGCGGCTTCACCTTTAGCAGCTACGCGATGAGCTGGGTG CGCCAGGCACCGGGTAAAGGTCTGGAATGGGTGAGCGCGATTAGCGGTAGCGGCG GCAGCACCTACTATGCGGATAGCGTGAAAGGCCGTTTTACCATCTCGCGTGATAACT CGAAAAACACCCTGTACCTGCAGATGAACAGCCTGCGTGCGGAAGATACCGCGGTG TATTATTGCGCACGTAAGTATCGTCCTTCTAGGGTGTTCGATTCCTGGGGTCAGGGCA CTCTGGTGACCGTGTCGAGCGCTAGCACCAAGGGCCCATCCGTCTTCCCCCTGGCG CCCTGCTCCAGGAGCACCTCCGAGAGCACAGCCGCCCTGGGCTGCCTGGTCAAGG ACTACTTCCCCGAACCGGTGACGGTGTCGTGGAACTCAGGCGCCCTGACCAGCGGC GTGCACACCTTCCCGGCTGTCCTACAGTCCTCAGGACTCTACTCCCTCAGCAGCGTG GTGACCGTGCCCTCCAGCAGCTTGGGCACGAAGACCTACACCTGCAACGTAGATCA CAAGCCCAGCAACACCAAGGTGGACAAGAGAGTTGAGTCCAAATATGGTCCCCCAT GCCCACCATGCCCAGCACCTGAGTTCCTGGGGGGACCATCAGTCTTCCTGTTCCCC CAAAACCCAAGGACACTCTCATGATCTCCCGGACCCCTGAGGTCACGTGCGTGGTG GTGGACGTGAGCCAGGAAGACCCCGAGGTCCAGTTCAACTGGTACGTGGATGGCG TGGAGGTGCATAATGCCAAGACAAAGCCGCGGGAGGAGCAGTTCAACAGCACGTA CCGTGTGGTCAGCGTCCTCACCGTCCTGCACCAGGACTGGCTGAACGGCAAGGAG TACAAGTGCAAGGTCTCCAACAAAGGCCTCCCGTCCTCCATCGAGAAAACCATCTC CAAAGCCAAAGGGCAGCCCCGAGAGCCACAGGTGTACACCCTGCCCCCATCCCAG GAGGAGATGACCAAGAACCAGGTCAGCCTGACCTGCCTGGTCAAAGGCTTCTACC CCAGCGACATCGCCGTGGAGTGGGAGAGCAATGGGCAGCCGGAGAACAACTACAA GACCACGCCTCCCGTGCTGGACTCCGACGGCTCCTTCTTCCTCTACAGCAGGCTAA CCGTGGACAAGAGCAGGTGGCAGGAGGGGAATGTCTTCTCATGCTCCGTGATGCAT GAGGCTCTGCACAACCACTACACACAGAAGAGCCTCTCCCTGTCCCTGGGTTGA (SEQ ID NO: 17) |
| Light Chain | Amino Acid | SYELTQPPSVSVAPGQTARITCSGDALGDKYASWYQQKPGQAPVLVIYEDSKRPSGIPE RFSGSNSGNTATLTISGTQAEDEADYYCQSNDADSVWVFGGGTKLTVLGQPKAAPSVT LFPPSSEELQANKATLVCLISDFYPGAVTVAWKADSSPVKAGVETTTPSKQSNNKYAAS SYLSLTPEQWKSHRSYSCQVTHEGSTVEKTVAPTECS (SEQ ID NO: 12) |
| | Coding Nucleotide | AGCTACGAACTGACCCAGCCGCCGAGCGTGTCGGTGGCGCCGGGTCAGACCGCGC GTATCACCTGCTCGGGCGATGCGCTGGGCGATAAATACGCGAGCTGGTATCAGCAG AAACCGGGTCAGGCACCGGTGCTGGTGATTTACGAAGATTCTAAACGCCCGTCTGG CATCCCGGAACGCTTTAGCGGCTCGAATTCGGGCAACACCGCGACCCTGACCATTA GCGGCACCCAGGCGGAGGATGAGGCGGACTATTACTGCCAGTCGAATGATGCCGAC TCGGTGTGGGTGTTTGGCGGTGGCACCAAGCTTACCGTCCTAGGTCAGCCCAAGGC TGCCCCCTCGGTCACTCTGTTCCCGCCCTCCTCTGAGGAGCTTCAAGCCAACAAGG CCACACTGGTGTGTCTCATAAGTGACTTCTACCCGGGAGCCGTGACAGTGGCCTGG AAGGCAGATAGCAGCCCCGTCAAGGCGGGAGTGGAGACCACCACACCCTCCAAAC AAAGCAACAACAAGTACGCGGCCAGCAGCTATCTGAGCCTGACGCCTGAGCAGTG GAAGTCCCACAGAAGCTACAGCTGCCAGGTCACGCATGAAGGGAGCACCGTGGAG AAGACAGTGGCCCCTACAGAATGTTCATAG (SEQ ID NO: 18) |

Note: In the amino acid sequences of the heavy/light chains above, the constant region sequences are located downstream of the sequences and designated by underlined double straight lines, and the complementarity-determining region (CDR) sequences are located in the middle of the sequences and indicated in bold.

### 2.5 Activity Analysis of Anti-S100A8/A9 Monoclonal Antibody

### 2.5.1 Experiment for Affinity Determination of Anti-S100A8/A9 Monoclonal Antibody by Reichert 4SPR

The anti-S100A8/A9 monoclonal antibody was covalently conjugated onto an SR7000 GOLD SENSOR SLIDE biosensor chip (Reichert, PART NO: 13206066). When target molecules flowed over the chip surface, they were captured by the anti-S100A8/A9 monoclonal antibody on the chip. The Reichert 4SPR instrument was used to detect the reaction signals in real time, thereby obtaining the association and dissociation curves of the antigen and the antibody. Affinity values were then derived by fitting the curves. In the present application, the Reichert 4SPR was used to further determine the affinity of the screened anti-S100A8/A9 monoclonal antibody for both the human heterodimeric antigen hS100A8/A9 and the monkey heterodimeric antigen cS100A8/A9. The results are shown in Table 2 below. The results demonstrate that the anti-S100A8/A9 monoclonal antibody screened in the present application exhibits the strongest affinity for hS100A8/A9, while its affinity for cS100A8/A9 is weaker than that for hS100A8/A9.

**Table 2. Affinity Determination Results**

| Antibody | Antigen | KD (M) |
|---|---|---|
| Anti-S100A8/A9 monoclonal antibody | hS100A8/A9 | (8.95E±0.54)-09 |
| Anti-S100A8/A9 monoclonal antibody | cS100A8/A9 | (2.28E±0.13)-08 |

### 2.5.2 Specificity of Anti-S100A8/A9 Monoclonal Antibody Against Homologous Family Proteins

The binding specificity of the anti-S100A8/A9 monoclonal antibody against homologous family proteins was assessed by ELISA. 96-well plates were coated with human S100 family proteins (S100A1, Uniprot: P23297; S100A2, Uniprot: P29034; S100A3, Uniprot: P33764; S100A4, Uniprot: P26447; S100A5, Uniprot: P33763; S100A6, Uniprot: P06703; S100A7, Uniprot: P31151; S100A8, Uniprot: P05109; S100A9, Uniprot: P06702; S100A10, Uniprot: P60903; S100A11, Uniprot: P31949; S100A12, Uniprot: P80511; S100A13, Uniprot: Q99584; S100A14, Uniprot: Q9HCY8; S100A15, Uniprot: Q86SG5; S100A16, Uniprot: Q96FQ6; S100A7L2, Uniprot: Q5SY68; S100B, Uniprot: P04271; S100G, Uniprot: P29377; S100P, Uniprot: P25815; S100Z, Uniprot: Q8WXG8; S100A8/A9) (0.5 µg/mL, 50 µL/well, overnight at 4°C). The next day, the liquids in the plates were discarded. The plates were blocked with a blocking buffer (4% milk-PBS) for 1 hour at 37°C. The anti-S100A8/A9 monoclonal antibody was added with a concentration of 20 µg/mL for binding in plates for 1 hour at 37°C. The plates were then washed four times with PBST, followed by addition of horseradish peroxidase-conjugated goat anti-human IgG (ZSGB-BIO, ZB-2304). The ELISA results demonstrate that the anti-S100A8/A9 monoclonal antibody prepared in the present application exhibits specific binding to hS100A8/A9, with no significant binding to other S100 family proteins (Figure 2).

### 2.5.3 Specificity of Anti-S100A8/A9 Monoclonal Antibody Against Other Unrelated Proteins

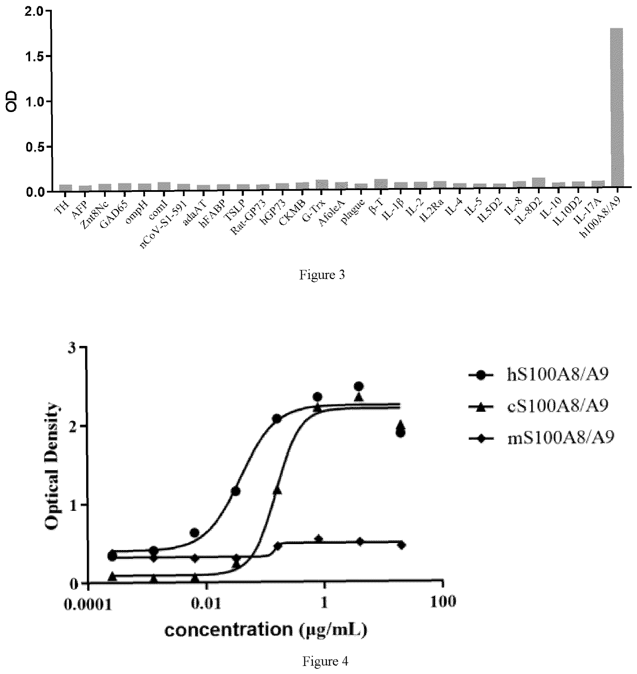

The binding specificity of the anti-S100A8/A9 monoclonal antibody against unrelated proteins was assessed by ELISA. 96-well plates were coated with other 28 random unrelated proteins (HT, Uniprot: P07911; AFP, Uniprot: P02771; Znt8Nc, Uniprot: Q8IWU4; GAD65, Uniprot: Q05329; ompH, Uniprot: A9KC33; coml, Uniprot: Q57333; nCov-S1-591, Uniprot: P0DTC2; adaAT, Uniprot: L0BY86; hFABP, Uniprot: P05413; TSLP, Uniprot: Q969D9; Rat-GP73, Uniprot: D4AEL2; hGP73, Uniprot: Q8NBJ4; CKMB, Uniprot: P06732+ P12277; G-Trx, Uniprot: P01350+P0AA25; AfoleA, Uniprot: G1XA82; plague, Uniprot: P26948; β-T, Uniprot: P05067+P0AA25; IL-1β, Uniprot: P01584; IL-2, Uniprot: P60568; IL2Ra, Uniprot: P01589; IL-4, Uniprot: P05112; IL-5, Uniprot: P05113; IL5D2, Uniprot: P05113; IL-8, Uniprot: P10145; IL-8D2, Uniprot: P10145; IL-10, Uniprot: P22301; IL-10D2, Uniprot: P22301; IL-17A, Uniprot: Q16552) (0.5 µg/mL, 50 µL/well, overnight at 4°C). The next day, the liquids in the plates were discarded. The plates were blocked with a blocking buffer (4% milk-PBS) for 1 hour at 37°C. The anti-S100A8/A9 monoclonal antibody was added with a concentration of 20 µg/mL for binding in plates for 1 hour at 37°C. The plates were then washed four times with PBST, followed by addition of horseradish peroxidase-conjugated goat anti-human IgG (ZSGB-BIO, ZB-2304). The ELISA results demonstrate that the anti-S100A8/A9 monoclonal antibody prepared in the present application exhibits specific binding to hS100A8/A9, with no significant binding to the random unrelated proteins (Figure 3).

### 2.5.4 Species Specificity Analysis of Anti-S100A8/A9 Monoclonal Antibody

The species specificity of the anti-S100A8/A9 monoclonal antibody was assessed by ELISA. 96-well plates were coated with the anti-S100A8/A9 monoclonal antibody (0.5 µg/mL, 50 µL/well, overnight at 4°C). The next day, the liquids in the plates were discarded. The plates were blocked with a blocking buffer (4% milk-PBS) for 1 hour at 37°C. S100A8/A9 antigens from 3 species (human heterodimeric antigen hS100A8/A9, mouse heterodimeric antigen mS100A8/A9, and monkey heterodimeric antigen cS100A8/A9) were added, respectively. The starting concentration of the S100A8/A9 antigens was 20 µg/mL, and 5-fold gradients were set, with 8 dilutions in total. Binding in plates was carried out for 1 hour at 37°C. The plates were then washed four times with PBST. HRP-conjugated 6×His, His-Tag Monoclonal antibody (Proteintech, Cat. No. HRP-66005) was used as a secondary antibody. The ELISA results demonstrate that the anti-S100A8/A9 monoclonal antibody screened in the present application exhibits the strongest affinity for hS100A8/A9, followed by cS100A8/A9, with no significant binding to mS100A8/A9 (Figure 4).

### 2.5.5 Stability Analysis of Anti-S100A8/A9 Monoclonal Antibody in Serum and PBS

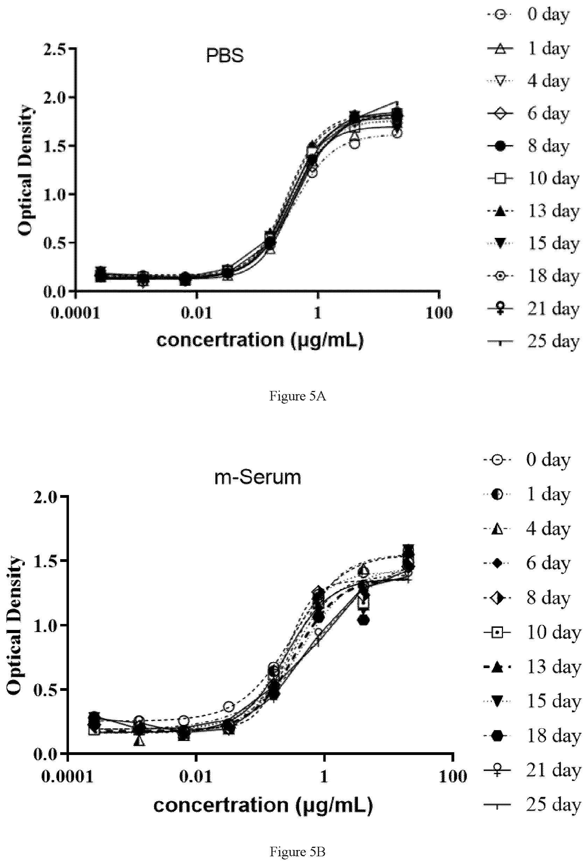

The stability of the anti-S100A8/A9 monoclonal antibody in PBS and C57BL/6 mouse serum (m-Serum) was further analyzed in the present application. The specific implementation methods were as follows: Filtered and sterilized anti-S100A8/A9 monoclonal antibody samples were diluted to 100 µg/mL in sterile PBS and mouse serum, with a volume of 200 µL/tube, and placed in a 37°C incubator for 0, 1, 4, 6, 8, 10, 13, 15, 18, 21, and 25 days. After 25 days, the samples in serum and PBS were analyzed by ELISA for binding to the hS100A8/A9 antigen (Figures 5A and 5B). The results demonstrate that the anti-hS100A8/A9 antibody obtained in the present application exhibits good antigen-binding stability in both C57BL/6 mouse serum and PBS.

### Example 3: Epitope Analysis of Binding of Anti-S100A8/A9 Monoclonal Antibody to hS100A8/A9

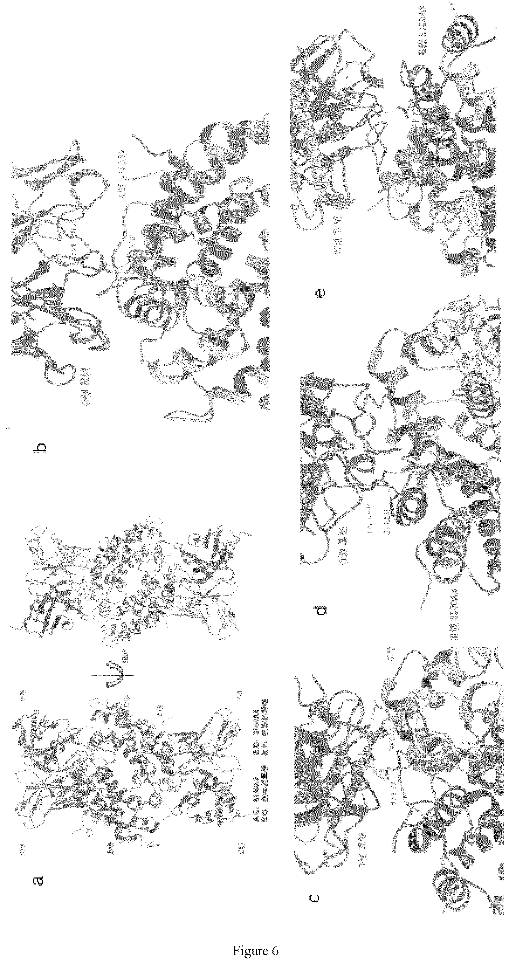

The antigen-antibody binding epitope was analyzed by cryo-electron microscopy. The implementation method was briefed as follows: S100A8/A9 samples were applied to glow-discharged Quantifoil Au R1.2/1.3 300-mesh grids. The micrographs were collected using a 300 kV Thermo Fisher Krios G4 cryo-electron microscope equipped with Falcon 4. The magnification was 96,000x, and the pixel size was 0.808. A total of 6,258 micrographs were collected, each with an electron dose of 50.2 e-Å-2 s-1 and a defocus range of -1.0 µm to -2.0 µm. The collected 6,258 good micrographs were subjected to further data processing using cryoSMART (Seemore Biotechnology Co., Ltd.). Particles were automatically picked via Blob-picker, followed by two rounds of 2D classification to pick out the best particles as templates for Template picking for three-dimensional reconstruction. A density map at 2.38 Å resolution was finally obtained. AlphaFold2 prediction was carried out based on amino acid sequences. The prediction results were fitted into the calculated map using UCSF Chimera. Subsequently, manual amino acid side chain adjustments were performed in COOT. Finally, the refined model was subjected to real-space refinement using PHENIX. The results (Figure 6) demonstrate that the core epitopes of the antigens are as follows: S100A8: D14, L21, and G24; S100A9: E60, K72, D98, E99, P101, G102, and H103. The antibody paratopes are as follows: Light chain: K52; Heavy chain: S54, S57, Y59, K99, R101, P102, and R104.

### Example 4: In-Vitro Bioactivity Assay of Anti-S100A8/A9 Monoclonal Antibody

### 4.1 Inhibitory effects of anti-S100A8/A9 monoclonal antibody on inflammatory cytokine release from hS100A8/A9-stimulated human PBMCs

The anti-S100A8/A9 monoclonal antibody was prepared at a starting concentration of 2,000 µg/mL, followed by 5-fold gradient dilutions, with 8 dilutions. Each dilution sample was added to 96-well plates (purchased from Corning, Cat. No. 3599), at 50 µL per well. The hS100A8/A9 protein was prepared at a concentration of 60 µg/mL, and added to 96-well plates at 50 µL per well for mixing with gradient concentrations of the anti-S100A8/A9 monoclonal antibody, respectively. Both the antigen and antibody used in this experiment contained pyrogen levels of less than 2 EU/mL.

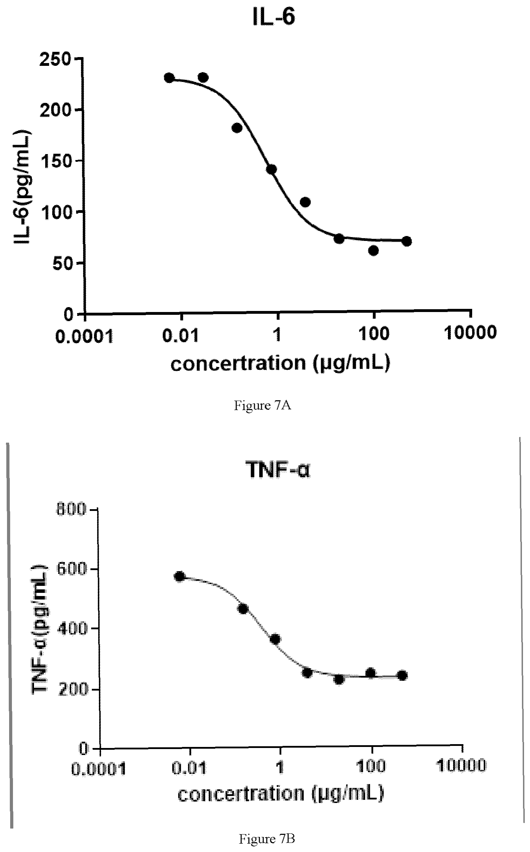

The ligand protein was mixed uniformly with the gradient-diluted antibody in equal volumes. Incubation was carried out for 30 minutes at room temperature. Human PBMCs (purchased from Milecell Biotechnologies, Cat. No. PB010C) were thawed, adjusted to a cell density of 1.0×10⁶ cells/mL, and applied to the corresponding positions of 96-well plates at 100 µL per well to be mixed uniformly with a drug. Meanwhile, the Blank control group (PBMC only) and the Positive control group (PBMC + S100A8/A9) were set. Medium volumes in the control groups were equalized. The cell plates were placed in a 37°C CO₂ incubator for incubation for approximately 20 hours prior to assay. All drugs, antigen dilutions and cell density adjustments were performed using RPMI 1640 complete medium (purchased from Thermo Fisher, Cat. No. A1049101) supplemented with 10% FBS (purchased from Thermo Fisher, Cat. No. 10099141C). After incubation, cell supernatants were collected, appropriately diluted, and assayed for cytokines using Human IL-6 ELISA Kit (purchased from Beijing Dakewe Biotech Co., Ltd., Cat. No. 1110603) and Human TNF-α ELISA Kit (purchased from Beijing Dakewe Biotech Co., Ltd., Cat. No. 1117203) according to assay instructions. OD450 values were read. Dose-response curves were drawn. The results demonstrate that the anti-S100A8/A9 monoclonal antibody in the present application can inhibit the binding of hS100A8/A9 to PBMC surface receptors, suppressing the release of inflammatory cytokines IL-6 (Figure 7A) and TNF-α (Figure 7B).

### 4.2 Inhibition of Proliferative Toxicity of hS100A8/A9 Toward Mouse Lymphoma EL-4 Cells by Anti-S100A8/A9 Monoclonal Antibody

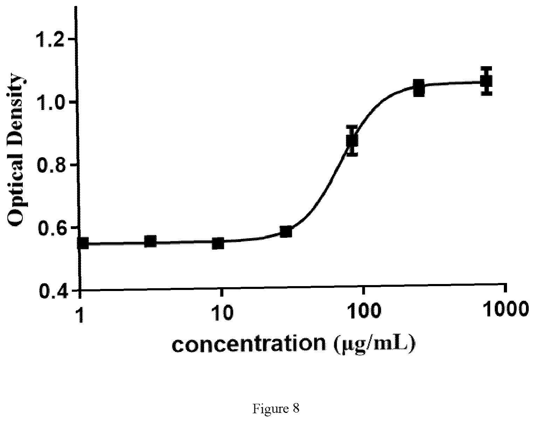

The hS100A8/A9 protein was prepared at a concentration of 50 µg/mL, and added to 96-well plates (purchased from Corning, Cat. No. 3599) at 50 µL per well. The density of EL-4 cells was adjusted to 1.0×10⁵ cells/mL and added to 96-well plates at 100 µL per well for uniform mixing with the hS100A8/A9 protein. Incubation was carried out for 6 hours in a 37°C CO₂ incubator. The anti-S100A8/A9 monoclonal antibody was prepared at a starting concentration of 9,240 µg/mL, followed by 3-fold gradient dilutions using an antibody diluent, with 8 dilutions in total. Each dilution sample was added to the corresponding positions of 96-well plates at 50 µL per well. All drug and antigen dilutions and cell density adjustments were performed using DMEM complete medium (purchased from Thermo Fisher, Cat. No. C11995500BT) supplemented with 10% horse serum (HS, purchased from EallBio, Cat. No. 03.16002A). Meanwhile, the Blank group (EL-4 only) and the Negative control group (EL-4 + S100A8/A9) were set. Medium volumes in the control groups were equalized. The cell plates were placed in a 37°C CO₂ incubator. After incubation for approximately 17 hours, CCK-8 reagent (purchased from EallBio, Cat. No. 03.17002DC) was added. After incubation for approximately 4 hours, OD450 was measured. Dose-response curves were drawn. The results (Figure 8) demonstrate that the anti-hS100A8/A9 antibody in the present application can bind to hS100A8/A9, block the proliferative toxicity of hS100A8/A9 toward mouse lymphoma EL-4 cells, and promote EL-4 cell proliferation, exhibiting a significant dose-dependent effect.

### Example 5: Blocking the Binding of hS100A8/A9 to Receptors by Anti-S100A8/A9 Monoclonal Antibody

### 5.1 Blocking the Binding of hS100A8/A9 to Cell Surface TLR4 or RAGE Receptors by Anti-S100A8/A9 Monoclonal Antibody

The anti-S100A8/A9 monoclonal antibody and an isotype control antibody (IgG) were prepared at a starting concentration of 800 µg/mL, followed by 3-fold gradient dilutions (for TLR4 receptor interaction) or 5-fold gradient dilutions (for RAGE receptor interaction) in an antibody diluent, with 10 or 8 dilutions in total. Each dilution sample was added to 96-well plates (purchased from Corning, Cat. No. 3894) at 50 µL per well. The hS100A8/A9 ligand protein was prepared at a concentration of 32 µg/mL (for TLR4 receptor interaction) or 8 µg/mL (for RAGE receptor interaction), and added to 96-well plates at 50 µL per well. The ligand protein was mixed uniformly with the gradient-diluted antibody in equal volumes. Incubation was carried out for 30 minutes at room temperature.

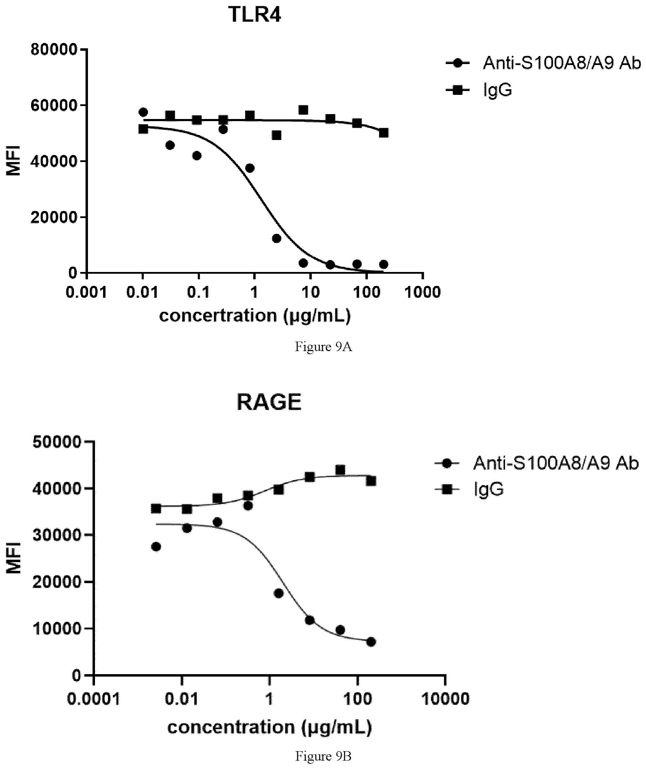

The HEK293-TLR4 engineered cell line was taken to detect binding of hS100A8/A9 to surface TLR4 receptors and blocking; the HEK293-RAGE engineered cell line was taken to detect binding of hS100A8/A9 to surface RAGE receptors and blocking. A single-cell suspension was obtained and counting was carried out. After centrifugation, cells were resuspended in PBS buffer and adjusted to a cell density of 2.0×10⁶ cells/mL. The cells were added to the corresponding positions of 96-well plates at 100 µL per well, and incubated for 1 hour at 4°C. After incubation, the cells were washed once by centrifugation at 3,000 rpm, and cell pellets were collected. 200 µL of PBS buffer was added per well. The cells were washed once by centrifugation at 3,000 rpm, and cell pellets were collected. FITC anti-His-Tag antibody prepared in advance (purchased from BioLegend, Cat. No. 362618) was added to the cell pellets at 100 µL per well, followed by incubation for 1 hour at 4°C. After the samples were taken out, PBS buffer was added at 100 µL per well, and cells were washed once at 3,000 rpm. The cells were resuspended in 200 µL of PBS buffer, and analyzed by flow cytometry, followed by collection of fluorescence signals collected in the FL1-A channel. The results demonstrate that the anti-hS100A8/A9 antibody in the present application can block the binding of hS100A8/A9 to engineered cell line surface TLR4 (Figure 9A) or RAGE (Figure 9B), exhibiting a significant dose-dependent effect. The isotype control antibody shows no blocking effect.

### 5.2 Blocking of Binding of hS100A8/A9 to PBMCs and THP-1 Cells by Anti-S100A8/A9 Monoclonal Antibody

The anti-S100A8/A9 monoclonal antibody was prepared at a starting concentration of 800 µg/mL, followed by 5-fold gradient dilutions (for PBMC interaction) or 4-fold gradient dilutions (for THP-1 cell interaction) in an antibody diluent, with 8 dilutions in total. Each dilution sample was added to 96-well plates (purchased from Corning, Cat. No. 3894) at 50 µL per well. The hS100A8/A9 ligand protein was prepared at a concentration of 50 µg/mL, and added to 96-well plates at 50 µL per well. The ligand protein was mixed uniformly with the gradient-diluted antibody in equal volumes. Incubation was carried out for 30 minutes at room temperature.

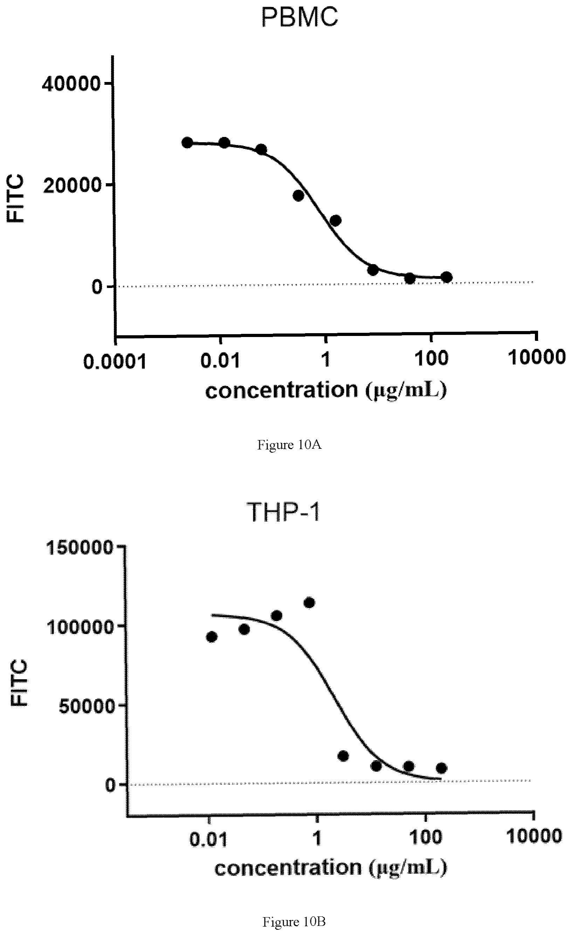

PBMCs and THP-1 cells were taken. A single-cell suspension was obtained, and counting was carried out. After centrifugation, cells were resuspended in PBS buffer and adjusted to a cell density of 2.0×10⁶ cells/mL. The cells were added to the corresponding positions of 96-well plates at 100 µL per well, and incubated for 1 hour at 4°C. After incubation, the cells were washed once by centrifugation at 3,000 rpm, and cell pellets were collected. 200 µL of PBS buffer was added per well. The cells were washed once by centrifugation at 3,000 rpm, and cell pellets were collected. FITC anti-His-Tag antibody prepared in advance (purchased from BioLegend, Cat. No. 362618) was added to the cell pellets at 100 µL per well, followed by incubation for 1 hour at 4°C. After the samples were taken out, PBS buffer was added at 100 µL per well, and cells were washed once at 3,000 rpm. The cells were resuspended in 200 µL of PBS buffer, and analyzed by flow cytometry, followed by collection of fluorescence signals collected in the FL1-A channel. The results demonstrate that the anti-S100A8/A9 monoclonal antibody in the present application can compete with hS100A8/A9 for binding to PBMCs (Figure 10A) and can also compete with hS100A8/A9 for binding to THP-1 cells (Figure 10B). The antibody exhibits a significant dose-dependent effect.

### Example 6: Activity of Genetically Engineered Mutants of Anti-S100A8/A9 Monoclonal Antibody

### 6.1 Binding Activity of CDR Single-Point Mutant Antibodies of Anti-S100A8/A9 Monoclonal Antibody

Alanine scanning and single-point directed mutation were carried out on 6 CDRs to obtain mutant antibodies with single-point mutations. These single-point mutant antibodies were expressed and purified using the method in 2.3 of Example 2. The binding activities of the purified mutant antibodies were compared with the binding activity of the parental antibody using the ELISA in 2.4 of Example 2. Screening revealed that, compared with the parental antibody (CDR sequences shown in SEQ ID Nos: 1, 2, 3, 4, 5, and 6, respectively), all mutant antibodies shown in Table 3 below retained the binding activity of the parental antibody.

### 6.2 Bioactivity of CDR Single-Point Mutant Antibodies of Anti-S100A8/A9 Monoclonal Antibody

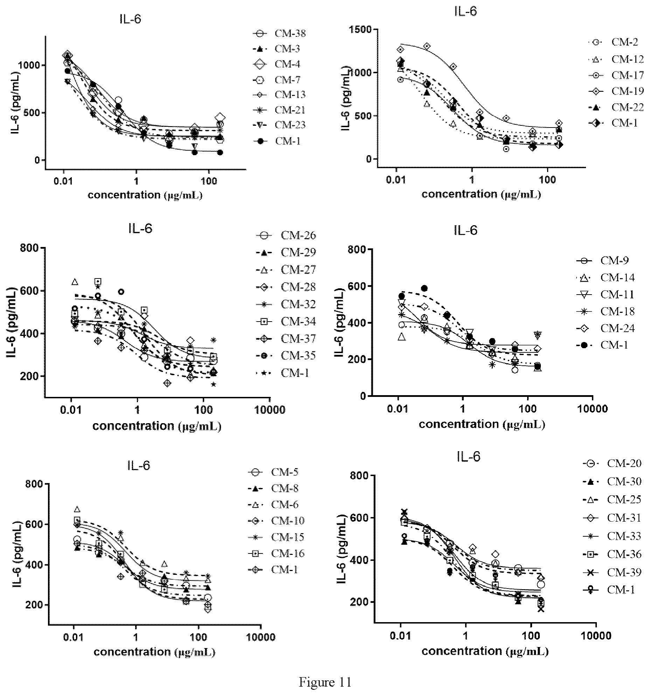

Using the method in 2.3 of Example 2, the 38 single-point mutant antibodies in Table 3 below were expressed and purified. The bioactivities of the single-point mutant antibodies were reflected by detecting their activities to inhibit IL-6 release from hS100A8/A9-activated human PBMCs. The specific detection methods were as follows: different mutants of the anti-S100A8/A9 monoclonal antibody were prepared at a starting concentration of 800 µg/mL, followed by 5-fold gradient dilutions in an antibody diluent, with 7 dilutions in total. Each dilution sample was added to 96-well plates (purchased from Corning, Cat. No. 3799), at 50 µL per well. The hS100A8/A9 ligand protein was prepared at a concentration of 80 µg/mL, and added to 96-well plates at 50 µL per well. Both the antigen and antibody used in this experiment contained pyrogen levels of less than 2 EU/mL. The ligand protein was mixed uniformly with the gradient-diluted antibody in equal volumes. Incubation was carried out for 30 minutes at room temperature. Human PBMCs (purchased from Milecell Biotechnologies, Cat. No. PB010C) were thawed, adjusted to a cell density of 1.0×10⁶ cells/mL, and applied to the corresponding positions of 96-well plates at 100 µL per well to be mixed uniformly with a preincubated mixture. Meanwhile, the Blank control group (PBMC only) and the Positive control group (PBMC + S100A8/A9) were set. Medium volumes in the control groups were equalized. The cell plates were placed in a 37°C CO₂ incubator for incubation for approximately 20 hours prior to assay. All antibody and antigen dilutions and cell density adjustments were performed using RPMI 1640 complete medium (purchased from Thermo Fisher, Cat. No. A1049101) supplemented with 10% FBS (purchased from Thermo Fisher, Cat. No. 10099141C). After incubation, cell supernatants were collected, appropriately diluted, and assayed for cytokines using Human IL-6 ELISA Kit (purchased from Beijing Dakewe Biotech Co., Ltd., Cat. No. 1110603) according to assay instructions. OD450 values were read. Dose-response curves were drawn. The results (Figure 11 and Table 3) demonstrate that all 38 mutants (shown in Table 3 below) of the anti-S100A8/A9 monoclonal antibody in the present application can inhibit binding of hS100A8/A9 to PBMC surface receptors and inhibit IL-6 release, exhibiting a significant dose-dependent effect.

The core sites in the HCDR were F29, Y32, A33, M34, Y59, A61, D62, Y100, S103, V105, F106, and S108. Mutants exhibiting approximately 10-fold enhanced cytological activity after mutations of core amino acid sites were: F29Y, F29W, Y59W, A61V, A61H, V105A, and F106Y. Mutants exhibiting approximately 2-fold enhanced cytological activity after site mutations were: F29H, A33V, Y100F, Y100H, and F106H. Mutants retaining higher cytological activity after mutations of other sites were: M34L, A61L, S103H, F106W, Y32H, Y32F, M34I, Y59F, D62A, D62H, S103T, and S108A. The HCDR sequences of antibodies exhibiting higher *in-vitro* bioactivities are shown in Table 3 below.

The core sites in the LCDR were Y31, E49, D50, S51, K52, A92, and Q88. The mutant exhibiting approximately 5-fold enhanced cytological activity after mutations of core amino acid sites was A92I. Mutants exhibiting approximately 2-fold enhanced cytological activity after site mutations were: Y31F and E49Q. Mutants exhibiting less than 2-fold enhanced cytological activity after site mutations were: K52H and A92L. Mutants retaining higher cytological activity after mutations of other sites were: D50Q, D50H, K52P, Q88D, S51A, S51H, K52R, A92V, and S94A. The LCDR sequences of antibodies exhibiting higher *in-vitro* bioactivities are shown in Table 3 below.

**Table 3 Changes in Cytological Activity of Antibodies After CDR Single-Point Mutations**

| Antibody | HCDR1, HCDR2, HCDR3, LCDR1, LCDR2, and LCDR3 sequences | IC50 (µg/ml) |
|---|---|---|
| CM-1 | SEQ ID Nos:1, 2, 3, 4, 5, 6 | 0.460 |
| CM-2 | SEQ ID Nos:24, 2, 3, 4, 5, 6 | 0.199 |
| CM-3 | SEQ ID Nos:25, 2, 3, 4, 5, 6 | 0.029 |
| CM-4 | SEQ ID Nos:26, 2, 3, 4, 5, 6 | 0.040 |
| CM-5 | SEQ ID Nos:29, 2, 3, 4, 5, 6 | 0.436 |
| CM-6 | SEQ ID Nos:30, 2, 3, 4, 5, 6 | 0.488 |
| CM-7 | SEQ ID Nos:31, 2, 3, 4, 5, 6 | 0.120 |
| CM-8 | SEQ ID Nos:32, 2, 3, 4, 5, 6 | 0.458 |
| CM-9 | SEQ ID Nos:33, 2, 3, 4, 5, 6 | 1.558 |
| CM-10 | SEQ ID Nos:1, 37, 3, 4, 5, 6 | 0.438 |
| CM-11 | SEQ ID Nos:1, 38, 3, 4, 5, 6 | 0.038 |
| CM-12 | SEQ ID Nos:1, 39, 3, 4, 5, 6 | 0.047 |
| CM-13 | SEQ ID Nos:1, 40, 3, 4, 5, 6 | 0.047 |
| CM-14 | SEQ ID Nos:1, 41, 3, 4, 5, 6 | 2.787 |
| CM-15 | SEQ ID Nos:1, 42, 3, 4, 5, 6 | 0.477 |
| CM-16 | SEQ ID Nos:1, 43, 3, 4, 5, 6 | 0.457 |
| CM-17 | SEQ ID Nos:1, 2, 49, 4, 5, 6 | 0.320 |
| CM-18 | SEQ ID Nos:1, 2, 50, 4, 5, 6 | 0.109 |
| CM-19 | SEQ ID Nos:1, 2, 51, 4, 5, 6 | 0.619 |
| CM-20 | SEQ ID Nos:1, 2, 52, 4, 5, 6 | 0.430 |
| CM-21 | SEQ ID Nos:1, 2, 53, 4, 5, 6 | 0.010 |
| CM-22 | SEQ ID Nos:1, 2, 54, 4, 5, 6 | 0.162 |
| CM-23 | SEQ ID Nos:1, 2, 55, 4, 5, 6 | 0.024 |
| CM-24 | SEQ ID Nos:1, 2, 56, 4, 5, 6 | 0.670 |
| CM-25 | SEQ ID Nos:1, 2, 57, 4, 5, 6 | 0.471 |
| CM-26 | SEQ ID Nos:1, 2, 3, 66, 5, 6 | 0.168 |
| CM-27 | SEQ ID Nos:1, 2, 3, 4, 70, 6 | 0.253 |
| CM-28 | SEQ ID Nos:1, 2, 3, 4, 71, 6 | 1.742 |
| CM-29 | SEQ ID Nos:1, 2, 3, 4, 72, 6 | 1.999 |
| CM-30 | SEQ ID Nos:1, 2, 3, 4, 73, 6 | 0.434 |
| CM-31 | SEQ ID Nos:1, 2, 3, 4, 74, 6 | 0.480 |
| CM-32 | SEQ ID Nos:1, 2, 3, 4, 75, 6 | 0.536 |
| CM-33 | SEQ ID Nos:1, 2, 3, 4, 76, 6 | 0.452 |
| CM-34 | SEQ ID Nos:1, 2, 3, 4, 77, 6 | 1.222 |
| CM-35 | SEQ ID Nos:1, 2, 3, 4, 5, 96 | 0.655 |
| CM-36 | SEQ ID Nos:1, 2, 3, 4, 5, 97 | 0.393 |
| CM-37 | SEQ ID Nos:1, 2, 3, 4, 5, 98 | 0.335 |
| CM-38 | SEQ ID Nos:1, 2, 3, 4, 5, 99 | 0.084 |
| CM-39 | SEQ ID Nos:1, 2, 3, 4, 5, 100 | 0.452 |

### 6.3 Binding Activity of CDR Combinatorial Mutant Antibodies of Anti-S100A8/A9 Monoclonal Antibody

In conjunction with the results in Examples 6.1 and 6.2, combinatorial mutations were carried out on multiple mutation sites in the CDR to obtain mutant antibodies with combinatorial mutations. The combinatorial mutant antibodies were expressed and purified using the method in 2.3 of Example 2. The binding activities of the purified combinatorial mutant antibodies were compared with the binding activity of the parental antibody using the ELISA in 2.4 of Example 2. Screening revealed that, compared with the parental antibody (CDR sequences shown in SEQ ID Nos: 1, 2, 3, 4, 5, and 6, respectively), all combinatorial mutant antibodies shown in Table 4 below retained the binding activity of the parental antibody.

### 6.4 Bioactivity of CDR Combinatorial Mutant Antibodies of Anti-S100A8/A9 Monoclonal Antibody

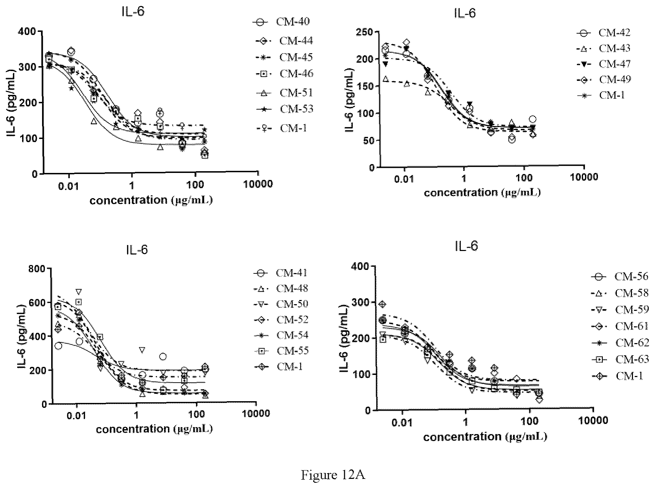

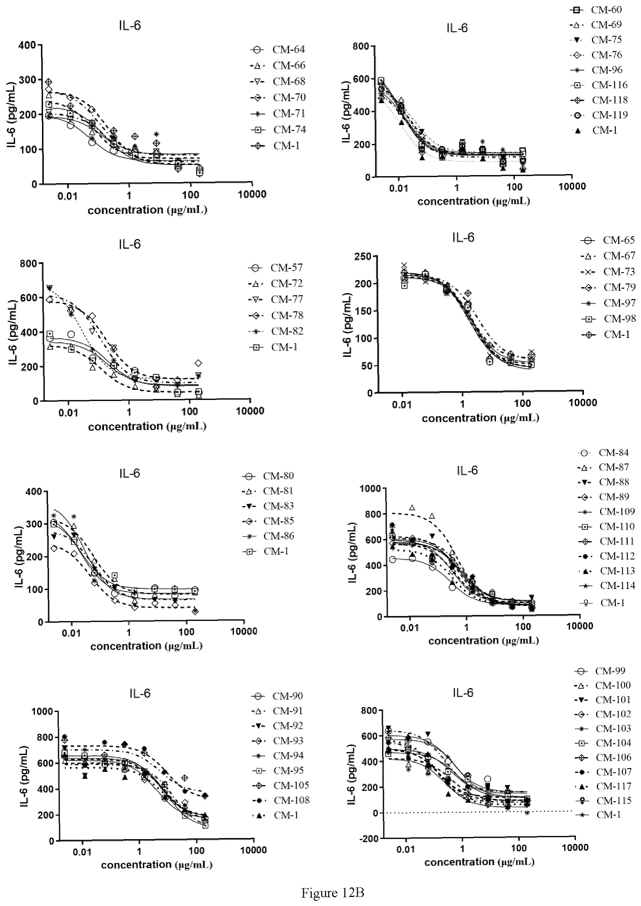

Using the method in 2.3 of Example 2, the 80 multi-point combinatorial mutant antibodies in Table 4 below were expressed and purified. The bioactivities of the multi-point combinatorial mutant antibodies were assayed for inhibition according to the method in 6.2 of Example 6. The results (Figure 12 and Table 4) demonstrate that all 80 combinatorial mutants (shown in Table 4 below) of the anti-S100A8/A9 monoclonal antibody in the present application can inhibit binding of hS100A8/A9 to PBMC surface receptors and inhibit IL-6 release, exhibiting a significant dose-dependent effect.

The core sites in the HCDR were SEQ ID NOs: 27-64. The HCDR sequences of antibodies exhibiting higher *in-vitro* bioactivities are shown in Table 4 below.

The core sites in the LCDR were SEQ ID NOs: 65-109. The LCDR sequences of antibodies exhibiting higher *in-vitro* bioactivities are shown in Table 4 below.

**Table 4 Changes in Cytological Activity of Antibodies After CDR Combinatorial Mutations**

| Antibody | HCDR1, HCDR2, HCDR3, LCDR1, LCDR2, and LCDR3 sequences | Relative IC50 (IC50 relative to CM-1) |
|---|---|---|
| CM-1 | SEQ ID Nos:1, 2, 3, 4, 5, 6 | 1 |
| CM-40 | SEQ ID Nos:1, 38, 3, 4, 5, 97 | 2.180 |
| CM-41 | SEQ ID Nos:1, 2, 57, 4, 5, 100 | 1.115 |
| CM-42 | SEQ ID Nos:1, 42, 57, 4, 5, 99 | 0.866 |
| CM-43 | SEQ ID Nos:1, 42, 57, 4, 73, 6 | 1.987 |
| CM-44 | SEQ ID Nos:1, 2, 57, 4, 73, 100 | 1.440 |
| CM-45 | SEQ ID Nos:1, 42, 3, 4, 73, 100 | 2.086 |
| CM-46 | SEQ ID Nos:1, 42, 53, 66, 5, 6 | 1.509 |
| CM-47 | SEQ ID Nos:25, 2, 53, 4, 5, 99 | 2.122 |
| CM-48 | SEQ ID Nos:1, 42, 57, 66, 5, 99 | 1.581 |
| CM-49 | SEQ ID Nos:1, 38, 57, 4, 73, 99 | 0.617 |
| CM-50 | SEQ ID Nos:1, 42, 57, 4, 73, 100 | 0.679 |
| CM-51 | SEQ ID Nos:1, 31, 57, 4, 76, 100 | 0.433 |
| CM-52 | SEQ ID Nos:25, 2, 53, 4, 73, 99 | 1.193 |
| CM-53 | SEQ ID Nos:1, 42, 53, 4, 73, 100 | 0.357 |
| CM-54 | SEQ ID Nos:25, 2, 53, 66, 5, 99 | 0.997 |
| CM-55 | SEQ ID Nos:1, 39, 53, 66, 5, 100 | 1.667 |
| CM-56 | SEQ ID Nos:1, 42, 57, 4, 73, 104 | 1.125 |
| CM-57 | SEQ ID Nos:1, 42, 57, 66, 73, 104 | 1.158 |
| CM-58 | SEQ ID Nos:1, 46, 57, 4, 73, 104 | 1.520 |
| CM-59 | SEQ ID Nos:1, 42, 64, 4, 73, 104 | 1.041 |
| CM-60 | SEQ ID Nos:1, 42, 63, 4, 73, 104 | 0.936 |
| CM-61 | SEQ ID Nos:25, 42, 57, 4, 85, 100 | 0.923 |
| CM-62 | SEQ ID Nos:31, 42, 57, 4, 87, 100 | 1.583 |
| CM-63 | SEQ ID Nos:1, 46, 57, 4, 73, 106 | 1.330 |
| CM-64 | SEQ ID Nos:1, 42, 63, 66, 73, 100 | 0.500 |
| CM-65 | SEQ ID Nos:25, 42, 57, 4, 85, 104 | 0.618 |
| CM-66 | SEQ ID Nos:25, 42, 63, 4, 73, 104 | 0.694 |
| CM-67 | SEQ ID Nos:29, 42, 62, 4, 87, 100 | 0.548 |
| CM-68 | SEQ ID Nos:32, 42, 63, 4, 73, 105 | 1.776 |
| CM-69 | SEQ ID Nos:1, 46, 63, 4, 85, 100 | 1.925 |
| CM-70 | SEQ ID Nos:1, 47, 63, 66, 73, 100 | 1.949 |
| CM-71 | SEQ ID Nos:1, 42, 63, 66, 73, 104 | 1.234 |
| CM-72 | SEQ ID Nos:25, 42, 63, 66, 73, 104 | 0.639 |
| CM-73 | SEQ ID Nos:25, 42, 63, 4, 85, 104 | 0.703 |
| CM-74 | SEQ ID Nos:1, 46, 64, 4, 87, 104 | 1.516 |
| CM-75 | SEQ ID Nos:1, 48, 63, 66, 85, 100 | 2.854 |
| CM-76 | SEQ ID Nos:1, 43, 63, 66, 73, 106 | 1.550 |
| CM-77 | SEQ ID Nos:1, 42, 57, 4, 88, 100 | 0.633 |
| CM-78 | SEQ ID Nos:1, 42, 57, 4, 89, 100 | 0.990 |
| CM-79 | SEQ ID Nos:1, 42, 57, 4, 90, 100 | 0.695 |
| CM-80 | SEQ ID Nos:1, 42, 57, 4, 92, 100 | 0.943 |
| CM-81 | SEQ ID Nos:1, 42, 57, 4, 91, 100 | 2.510 |
| CM-82 | SEQ ID Nos:1, 42, 57, 4, 94, 100 | 0.156 |
| CM-83 | SEQ ID Nos:1, 42, 57, 4, 93, 100 | 2.481 |
| CM-84 | SEQ ID Nos:1, 42, 57, 4, 93, 109 | 0.393 |
| CM-85 | SEQ ID Nos:23, 42, 57, 4, 73, 100 | 1.864 |
| CM-86 | SEQ ID Nos:29, 42, 57, 4, 73, 100 | 1.273 |
| CM-87 | SEQ ID Nos:27, 42, 57, 4, 73, 108 | 0.749 |
| CM-88 | SEQ ID Nos:27, 42, 57, 4, 73, 107 | 1.014 |
| CM-89 | SEQ ID Nos:27, 42, 57, 4, 73, 109 | 0.572 |
| CM-90 | SEQ ID Nos:27, 42, 57, 65, 73, 100 | 1.545 |
| CM-91 | SEQ ID Nos:27, 42, 57, 69, 73, 100 | 1.099 |
| CM-92 | SEQ ID Nos:27, 42, 57, 65, 73, 109 | 0.697 |
| CM-93 | SEQ ID Nos:27, 42, 57, 4, 93, 109 | 0.953 |
| CM-94 | SEQ ID Nos:27, 42, 57, 65, 86, 100 | 2.812 |
| CM-95 | SEQ ID Nos:27, 42, 57, 4, 86, 100 | 1.114 |
| CM-96 | SEQ ID Nos:27, 42, 57, 67, 73, 108 | 1.474 |
| CM-97 | SEQ ID Nos:27, 42, 57, 4, 95, 108 | 0.599 |
| CM-98 | SEQ ID Nos:27, 42, 57, 4, 94, 108 | 0.703 |
| CM-99 | SEQ ID Nos:27, 45, 57, 4, 73, 100 | 1.305 |
| CM-100 | SEQ ID Nos:27, 45, 57, 65, 73, 100 | 1.121 |
| CM-101 | SEQ ID Nos:27, 45, 57, 68, 73, 100 | 0.683 |
| CM-102 | SEQ ID Nos:27, 45, 57, 4, 73, 108 | 0.464 |
| CM-103 | SEQ ID Nos:27, 45, 57, 4, 73, 107 | 0.687 |
| CM-104 | SEQ ID Nos:27, 45, 57, 4, 73, 109 | 1.761 |
| CM-105 | SEQ ID Nos:27, 45, 57, 69, 73, 100 | 1.497 |
| CM-106 | SEQ ID Nos:27, 45, 57, 65, 73, 109 | 0.352 |
| CM-107 | SEQ ID Nos:27, 45, 57, 4, 86, 100 | 0.386 |
| CM-108 | SEQ ID Nos:27, 45, 57, 4, 93, 109 | 1.500 |
| CM-109 | SEQ ID Nos:27, 45, 57, 65, 86, 100 | 0.475 |
| CM-110 | SEQ ID Nos:34, 42, 57, 65, 73, 100 | 0.878 |
| CM-111 | SEQ ID Nos:1, 44, 57, 68, 73, 100 | 1.076 |
| CM-112 | SEQ ID Nos:1, 45, 57, 65, 73, 100 | 0.738 |
| CM-113 | SEQ ID Nos:1, 45, 57, 69, 73, 100 | 0.442 |
| CM-114 | SEQ ID Nos:1, 45, 57, 4, 86, 100 | 0.899 |
| CM-115 | SEQ ID Nos:28, 42, 57, 4, 73, 108 | 1.344 |
| CM-116 | SEQ ID Nos:27, 42, 59, 4, 73, 108 | 0.986 |
| CM-117 | SEQ ID Nos:27, 42, 58, 4, 73, 108 | 0.367 |
| CM-118 | SEQ ID Nos:27, 42, 60, 4, 73, 108 | 1.630 |
| CM-119 | SEQ ID Nos:27, 42, 61, 4, 73, 108 | 1.374 |

### Example 7: Pharmacokinetics Assay of Anti-S100A8/A9 Monoclonal Antibody

To investigate the metabolic profile of the anti-S100A8/A9 monoclonal antibody in the present application in mice, 5 male C57BL/6 mice were selected. Administration was carried out via tail vein injection at a dose of 10 mg/kg. Blood samples were collected at the following pharmacokinetic (PK) blood collection points: 5 min, 2 hours, 4 hours, 8 hours, 1 day, 3 days, 7 days, and 14 days after administration for serum antibody concentration assay.

The serum samples from the PK blood collection points were assayed for total antibody and active antibody concentrations using ELISA. A goat anti-human Fc antibody (1:2000, Sigma-Aldrich, Product number: I8885) and an antigen (1 µg/mL, hS100A8/A9) were diluted in PBS at 100 µL/well. Coating was carried out overnight at 4°C. 3% skim milk (diluted in PBS) was added to the well plates at 300 µL/well for blocking for 2 hours at 37°C. The known concentrations of antibody standards and the mouse serum were added to the well plates at 100 µL/well, respectively, for incubation for 1 hour at 37°C. The plates were washed. Horseradish peroxidase-conjugated goat anti-human IgG (H+L) (1:5000, Beijing TransGen Biotech Co., Ltd., Cat. No: ZB-2304) was added at 100 µL/well for incubation for 45 minutes at 37°C. The plates were washed again. TMB was added for color development for 10 minutes. Finally, a stop buffer was added. The optical density was measured at 450 nm using a microplate reader. The standard curve was fit. The sample concentrations were calculated by interpolating the standard curve. Pharmacokinetic parameters were calculated for 5 mice using Phoenix WinNonlin.

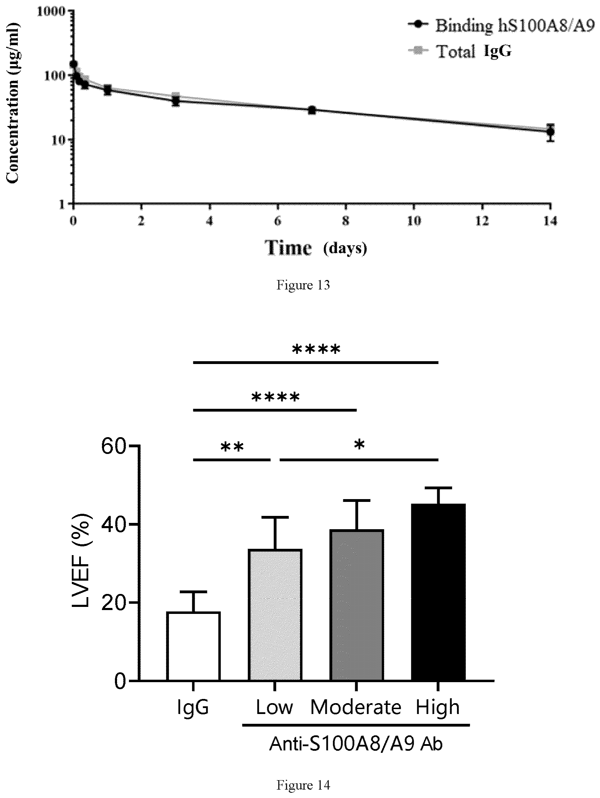

The results (Figure 13) demonstrate that the anti-S100A8/A9 monoclonal antibody in the present application exhibits an average half-life (t1/2) of 6.6 days in the 5 male C57BL/6 mice.

### Example 8: Improvement Effects of Anti-S100A8/A9 Monoclonal Antibody on Cardiac Injury and Cardiac Function in S100A8/A9 Humanized Mice with Acute Myocardial Infarction and Myocardial Ischemia/Reperfusion Injury

### 8.1 Improvement Effects of Anti-S100A8/A9 Monoclonal Antibody at Different Administration Doses on Cardiac Injury and Cardiac Function in S100A8/A9 Humanized Mice with Acute Myocardial Infarction

To investigate the dose-dependent improvement effects of the anti-S100A8/A9 monoclonal antibody in the present application on acute myocardial infarction (AMI) in mice, 40 S100A8/A9 humanized mice were selected and randomly divided into 4 groups: IgG Negative Control Group, Anti-S100A8/A9 Monoclonal Antibody Low-Dose Group, Anti-S100A8/A9 Monoclonal Antibody Moderate-Dose Group, and Anti-S100A8/A9 Monoclonal Antibody High-Dose Group, with 10 mice per group, with half male and half female. All mice underwent the left coronary artery ligation to establish AMI models. Some mice died during the surgery, and administration was carried out on 8 mice per group ultimately. Immediately after the myocardial infarction surgery, the anti-S100A8/A9 monoclonal antibody or IgG control was administered via angular vein injection. The low, moderate, and high doses of the anti-S100A8/A9 monoclonal antibody were 12 mg/kg, 16 mg/kg, and 24 mg/kg, respectively. The IgG administration dose was 24 mg/kg. The statuses of the mice were observed daily. At 14 days after myocardial infarction, the cardiac function was assessed in mice via echocardiography, and the left ventricular ejection fraction (LVEF) was calculated. The mice were euthanized, cardiac tissues were harvested, fixed, paraffin-embedded, sectioned, and subjected to Masson's staining. The infarct size was measured and calculated.

The results demonstrate that compared with the IgG negative control group, the low-dose, moderate-dose, and high-dose anti-S100A8/A9 monoclonal antibodies all can increase LVEF in mice after myocardial infarction, and the difference has statistical significance (p<0.05); compared with the low-dose anti-S100A8/A9 monoclonal antibody group, the high-dose group exhibits elevated LVEF, and the difference has statistical significance (p<0.05) (Figure 14).

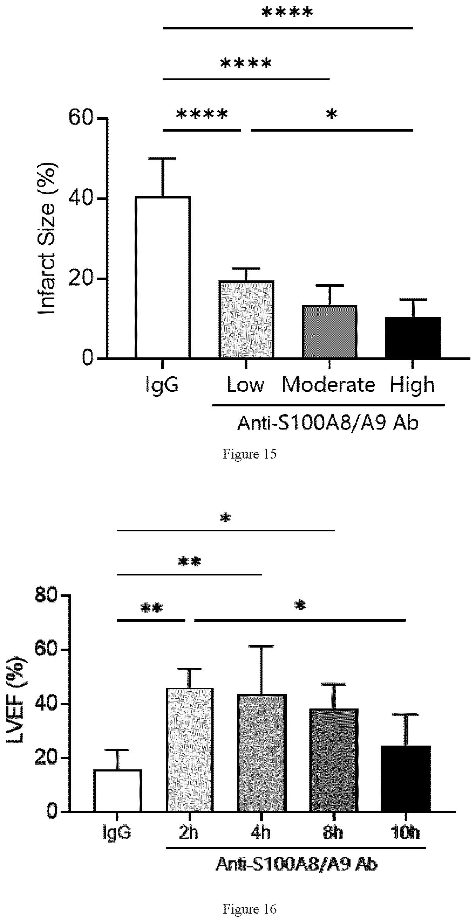

Compared with the IgG negative control group, the low-dose, moderate-dose, and high-dose anti-S100A8/A9 monoclonal antibodies all can reduce the cardiac infarct size, and the difference has statistical significance (p<0.05); compared with the low-dose anti-S100A8/A9 monoclonal antibody group, the high-dose group exhibits reduced cardiac infarct size, and the difference has statistical significance (p<0.05) (Figure 15).

### 8.2 Improvement Effects of Anti-S100A8/A9 Monoclonal Antibody at Different Administration Time Points on Cardiac Injury and Cardiac Function in S100A8/A9 Humanized Mice with Acute Myocardial Infarction

To investigate the therapeutic time window for the improvement effects of the anti-S100A8/A9 monoclonal antibody in the present application on AMI in mice, 50 hS100A8/A9 humanized mice were selected and randomly divided into 5 groups: IgG Negative Control Group, Anti-S100A8/A9 Monoclonal Antibody 2-Hour (h) Post-Surgery Administration Group, Anti-S100A8/A9 Monoclonal Antibody 4-h Post-Surgery Administration Group, Anti-S100A8/A9 Monoclonal Antibody 8-h Post-Surgery Administration Group, and Anti-S100A8/A9 Monoclonal Antibody 10-h Post-Surgery Administration Group, with half male and half female. All mice underwent left coronary artery ligation to establish AMI models. Some mice died during the myocardial infarction surgery. Administration was carried out on the remaining mice: 9 mice in the IgG group, 7 mice in the 2-h group, 7 mice in the 4-h group, 8 mice in the 8-h group, and 8 mice in the 10-h group. In the IgG negative control group, 2 hours after the myocardial infarction surgery, IgG control was administered via retro-orbital vein injection at a dose of 20 mg/kg. In the remaining 4 groups, the anti-S100A8/A9 monoclonal antibody was administered 2, 4, 8, and 10 hours after surgery, respectively, at a dose of 20 mg/kg. At 14 days after myocardial infarction, the cardiac function was assessed in mice via echocardiography, and the left ventricular ejection fraction (LVEF) was calculated. The mice were euthanized, cardiac tissues were harvested, fixed, paraffin-embedded, sectioned, and subjected to Masson's staining. The infarct size was measured and calculated.

The results demonstrate that compared with the IgG negative control group, administration of the anti-S100A8/A9 monoclonal antibody at 2, 4, and 8 hours after surgery all can increase LVEF in mice after myocardial infarction, and the difference has statistical significance (p<0.05); compared with the IgG negative control group, the 10-h post-surgery administration group exhibits no improvement in LVEF; compared with the 10-hour group, the 2-hour post-surgery administration group exhibits elevated LVEF, and the difference has statistical significance (p<0.05) (Figure 16).

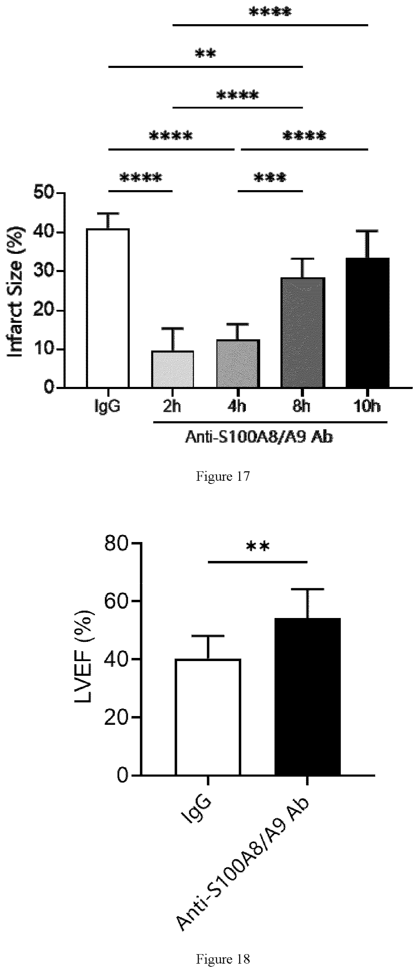

Compared with the IgG negative control group, administration of the anti-S100A8/A9 monoclonal antibody at 2, 4, and 8 hours after surgery all can reduce cardiac infarct size in mice after myocardial infarction, and the difference has statistical significance (p<0.05); compared with the IgG negative control group, the 10-h post-surgery group exhibits no significant improvement in cardiac infarct size; compared with the 8-h group, the 2-h and 4-h post-surgery administration groups both exhibit reduced cardiac infarct size, and the difference has statistical significance (p<0.05); compared with the 10-h group, the 2-h and 4-h post-surgery administration groups both exhibit reduced cardiac infarct size, and the difference has statistical significance (p<0.05) (Figure 17).

The results demonstrate that the therapeutic time window for administration of the anti-S100A8/A9 monoclonal antibody after myocardial infarction is within 8 hours.

### 8.3 Improvement Effects of Anti-S100A8/A9 Monoclonal Antibody on Cardiac Function in S100A8/A9 Humanized Mice after Myocardial Ischemia/Reperfusion

To investigate the improvement effects of the anti-S100A8/A9 monoclonal antibody in the present application on the cardiac function in mice after myocardial infarction/vascular recanalization, 20 S100A8/A9 humanized mice were selected and randomly divided into 2 groups: IgG Negative Control Group, and Anti-S100A8/A9 Monoclonal Antibody Treatment Group, with 10 mice per group, with half male and half female. All mice underwent left coronary artery ligation for 60 minutes, followed by ligation release to achieve vascular recanalization, simulating post-myocardial infarction vascular recanalization in humans after thrombolysis or PCI surgery. Some mice died or modeling was unsuccessful during the surgery. Administration was carried out on the remaining mice: 7 mice in the IgG negative control group, and 9 mice in the anti-S100A8/A9 monoclonal antibody group. The anti-S100A8/A9 monoclonal antibody or IgG was administered immediately upon vascular recanalization at a dose of 20 mg/kg. The statuses of the mice were observed daily after surgery. At 28 days after administration, the cardiac function was assessed in mice via echocardiography, and the LVEF was calculated.

The results demonstrate that compared with the IgG negative control group, the anti-S100A8/A9 monoclonal antibody can significantly increase the LVEF in mice after cardiac ischemia/reperfusion (p<0.05), and improve the cardiac function (Figure 18).

It should be stated that the above are only preferred embodiments of the present application and are not intended to limit the present application. For those skilled in the art, the present application can have various alterations and changes. Although specific implementations have been described, there may exist or may now be unforeseen alternatives, modifications, variations, improvements, and substantial equivalents to the above-described implementations for applicants or others skilled in the art. Therefore, the appended claims as submitted and claims that may be amended are intended to cover all such alternatives, modifications, variations, improvements, and substantial equivalents. Importantly, as technology continues to evolve, many elements described herein may be replaced by equivalent elements that emerge after the present application.

## Claims

1. A fully human antibody or antigen-binding fragment thereof, wherein complementarity-determining regions (CDRs) thereof defined according to a KABAT system have the following amino acid sequences:
an amino acid sequence of heavy chain CDR1 comprises GX₁TX₂SX₃X₄X₅X₆X₇, wherein X₁ is For Y, X₂ is F, H, Y, or W, X₃ is S or D, X₄ is Y, H, or F, X₅ is A or V, X₆ is M, I, or L, and X₇ is S or A;
an amino acid sequence of heavy chain CDR2 comprises AISGX₈GGSX₉X₁₀YX₁₁X₁₂SVKG, wherein X₈ is S or H, X₉ is Tor H, X₁₀ is Y, W, or F, X₁₁ is A, H, V, or L, and X₁₂ is D, A, or H;
an amino acid sequence of heavy chain CDR3 comprises KX₁₃RPX₁₄RX₁₅X₁₆DX₁₇, wherein X₁₃ is Y, F, or H, X₁₄ is S, T, or H, X₁₅ is V or A, X₁₆ is F, Y, H, or W, and X₁₇ is S, A, F, C, H, or Y;
an amino acid sequence of light chain CDR1 comprises SGDALX₁₈DKX₁₉X₂₀X₂₁, wherein X₁₈ is G or H, X₁₉ is Y or F, X₂₀ is A or M, and X₂₁ is S or A;
an amino acid sequence of light chain CDR2 comprises X₂₂X₂₃X₂₄X₂₅RX₂₆X₂₇, wherein X₂₂ is E or Q, X₂₃ is D, H, or Q, X₂₄ is S, A, or H, X₂₅ is K, H, R, P, or N, X₂₆ is P, Q, D, or N, and X₂₇ is S, D, Y, or W; and
an amino acid sequence of light chain CDR3 comprises X₂₈SNDX₂₉DX₃₀X₃₁W, wherein X₂₈ is Q or D, X₂₉ is A, V, I, or L, X₃₀ is S or A, and X₃₁ is V, Y, W, or R.

2. The antibody or antigen-binding fragment thereof according to claim 1, wherein the CDRs thereof have the following amino acid sequences:
the heavy chain CDR1 is selected from at least one of amino acid sequences shown in SEQ ID NOs: 1 and 23-34;
the heavy chain CDR2 is selected from at least one of amino acid sequences shown in SEQ ID NOs: 2 and 35-48;
the heavy chain CDR3 is selected from at least one of amino acid sequences shown in SEQ ID NOs: 3 and 49-64;
the light chain CDR1 is selected from at least one of amino acid sequences shown in SEQ ID NOs: 4 and 65-69;
the light chain CDR2 is selected from at least one of amino acid sequences shown in SEQ ID NOs: 5 and 70-95; and
the light chain CDR3 is selected from at least one of amino acid sequences shown in SEQ ID NOs: 6 and 96-109.

3. The antibody or antigen-binding fragment thereof according to claim 1 or 2, wherein the amino acid sequences of the CDRs thereof are selected from at least one of the following groups (1)-(119):
(1) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 6, respectively;
(2) the heavy chain CDR1, the heavy chain CDR2, the heavy chain CDR3, the light chain CDR1, the light chain CDR2, and the light chain CDR3 are amino acid sequences shown in SEQ ID NOs: 24, 2, 3, 4, 5, and 6, respectively;
(3) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 3, 4, 5, and 6, respectively;
(4) the heavy chain CDR1, the heavy chain CDR2, the heavy chain CDR3, the light chain CDR1, the light chain CDR2, and the light chain CDR3 are amino acid sequences shown in SEQ ID NOs: 26, 2, 3, 4, 5, and 6, respectively;
(5) the heavy chain CDR1, the heavy chain CDR2, the heavy chain CDR3, the light chain CDR1, the light chain CDR2, and the light chain CDR3 are amino acid sequences shown in SEQ ID NOs: 29, 2, 3, 4, 5, and 6, respectively;
(6) the heavy chain CDR1, the heavy chain CDR2, the heavy chain CDR3, the light chain CDR1, the light chain CDR2, and the light chain CDR3 are amino acid sequences shown in SEQ ID NOs: 30, 2, 3, 4, 5, and 6, respectively;
(7) the heavy chain CDR1, the heavy chain CDR2, the heavy chain CDR3, the light chain CDR1, the light chain CDR2, and the light chain CDR3 are amino acid sequences shown in SEQ ID NOs: 31, 2, 3, 4, 5, and 6, respectively;
(8) the heavy chain CDR1, the heavy chain CDR2, the heavy chain CDR3, the light chain CDR1, the light chain CDR2, and the light chain CDR3 are amino acid sequences shown in SEQ ID NOs: 32, 2, 3, 4, 5, and 6, respectively;
(9) the heavy chain CDR1, the heavy chain CDR2, the heavy chain CDR3, the light chain CDR1, the light chain CDR2, and the light chain CDR3 are amino acid sequences shown in SEQ ID NOs: 33, 2, 3, 4, 5, and 6, respectively;
(10) the heavy chain CDR1, the heavy chain CDR2, the heavy chain CDR3, the light chain CDR1, the light chain CDR2, and the light chain CDR3 are amino acid sequences shown in SEQ ID NOs: 1, 37, 3, 4, 5, and 6, respectively;
(11) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 3, 4, 5, and 6, respectively;
(12) the heavy chain CDR1, the heavy chain CDR2, the heavy chain CDR3, the light chain CDR1, the light chain CDR2, and the light chain CDR3 are amino acid sequences shown in SEQ ID NOs: 1, 39, 3, 4, 5, and 6, respectively;
(13) the heavy chain CDR1, the heavy chain CDR2, the heavy chain CDR3, the light chain CDR1, the light chain CDR2, and the light chain CDR3 are amino acid sequences shown in SEQ ID NOs: 1, 40, 3, 4, 5, and 6, respectively;
(14) the heavy chain CDR1, the heavy chain CDR2, the heavy chain CDR3, the light chain CDR1, the light chain CDR2, and the light chain CDR3 are amino acid sequences shown in SEQ ID NOs: 1, 41, 3, 4, 5, and 6, respectively;
(15) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 3, 4, 5, and 6, respectively;
(16) the heavy chain CDR1, the heavy chain CDR2, the heavy chain CDR3, the light chain CDR1, the light chain CDR2, and the light chain CDR3 are amino acid sequences shown in SEQ ID NOs: 1, 43, 3, 4, 5, and 6, respectively;
(17) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 49, 4, 5, and 6, respectively;
(18) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 50, 4, 5, and 6, respectively;
(19) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 51, 4, 5, and 6, respectively;
(20) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 52, 4, 5, and 6, respectively;
(21) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 53, 4, 5, and 6, respectively;
(22) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 54, 4, 5, and 6, respectively;
(23) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 55, 4, 5, and 6, respectively;
(24) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 56, 4, 5, and 6, respectively;
(25) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 5, and 6, respectively;
(26) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 66, 5, and 6, respectively;
(27) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 70, and 6, respectively;
(28) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 71, and 6, respectively;
(29) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 72, and 6, respectively;
(30) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 73, and 6, respectively;
(31) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 74, and 6, respectively;
(32) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 75, and 6, respectively;
(33) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 76, and 6, respectively;
(34) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 77, and 6, respectively;
(35) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 96, respectively;
(36) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 97, respectively;
(37) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 98, respectively;
(38) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 99, respectively;
(39) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 3, 4, 5, and 100, respectively;
(40) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 3, 4, 5, and 97, respectively;
(41) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 5, and 100, respectively;
(42) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 5, and 99, respectively;
(43) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 6, respectively;
(44) the heavy chain CDR1, the heavy chain CDR2, the heavy chain CDR3, the light chain CDR1, the light chain CDR2, and the light chain CDR3 are amino acid sequences shown in SEQ ID NOs: 1, 2, 57, 4, 73, and 100, respectively;
(45) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 3, 4, 73, and 100, respectively;
(46) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 53, 66, 5, and 6, respectively;
(47) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 4, 5, and 99, respectively;
(48) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 66, 5, and 99, respectively;
(49) the heavy chain CDR1, the heavy chain CDR2, the heavy chain CDR3, the light chain CDR1, the light chain CDR2, and the light chain CDR3 are amino acid sequences shown in SEQ ID NOs: 1, 38, 57, 4, 73, and 99, respectively;
(50) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 100, respectively;
(51) the heavy chain CDR1, the heavy chain CDR2, the heavy chain CDR3, the light chain CDR1, the light chain CDR2, and the light chain CDR3 are amino acid sequences shown in SEQ ID NOs: 1, 31, 57, 4, 76, and 100, respectively;
(52) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 4, 73, and 99, respectively;
(53) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 53, 4, 73, and 100, respectively;
(54) the heavy chain CDR1, the heavy chain CDR2, the heavy chain CDR3, the light chain CDR1, the light chain CDR2, and the light chain CDR3 are amino acid sequences shown in SEQ ID NOs: 25, 2, 53, 66, 5, and 99, respectively;
(55) the heavy chain CDR1, the heavy chain CDR2, the heavy chain CDR3, the light chain CDR1, the light chain CDR2, and the light chain CDR3 are amino acid sequences shown in SEQ ID NOs: 1, 39, 53, 66, 5, and 100, respectively;
(56) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 73, and 104, respectively;
(57) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 66, 73, and 104, respectively;
(58) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 57, 4, 73, and 104, respectively;
(59) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 64, 4, 73, and 104, respectively;
(60) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 4, 73, and 104, respectively;
(61) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 57, 4, 85, and 100, respectively;
(62) the heavy chain CDR1, the heavy chain CDR2, the heavy chain CDR3, the light chain CDR1, the light chain CDR2, and the light chain CDR3 are amino acid sequences shown in SEQ ID NOs: 31, 42, 57, 4, 87, and 100, respectively;
(63) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 57, 4, 73, and 106, respectively;
(64) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 66, 73, and 100, respectively;
(65) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 57, 4, 85, and 104, respectively;
(66) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 4, 73, and 104, respectively;
(67) the heavy chain CDR1, the heavy chain CDR2, the heavy chain CDR3, the light chain CDR1, the light chain CDR2, and the light chain CDR3 are amino acid sequences shown in SEQ ID NOs: 29, 42, 62, 4, 87, and 100, respectively;
(68) the heavy chain CDR1, the heavy chain CDR2, the heavy chain CDR3, the light chain CDR1, the light chain CDR2, and the light chain CDR3 are amino acid sequences shown in SEQ ID NOs: 32, 42, 63, 4, 73, and 105, respectively;
(69) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 63, 4, 85, and 100, respectively;
(70) the heavy chain CDR1, the heavy chain CDR2, the heavy chain CDR3, the light chain CDR1, the light chain CDR2, and the light chain CDR3 are amino acid sequences shown in SEQ ID NOs: 1, 47, 63, 66, 73, and 100, respectively;
(71) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 63, 66, 73, and 104, respectively;
(72) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 66, 73, and 104, respectively;
(73) the heavy chain CDR1, the heavy chain CDR2, the heavy chain CDR3, the light chain CDR1, the light chain CDR2, and the light chain CDR3 are amino acid sequences shown in SEQ ID NOs: 25, 42, 63, 4, 85, and 104, respectively;
(74) the heavy chain CDR1, the heavy chain CDR2, the heavy chain CDR3, the light chain CDR1, the light chain CDR2, and the light chain CDR3 are amino acid sequences shown in SEQ ID NOs: 1, 46, 64, 4, 87, and 104, respectively;
(75) the heavy chain CDR1, the heavy chain CDR2, the heavy chain CDR3, the light chain CDR1, the light chain CDR2, and the light chain CDR3 are amino acid sequences shown in SEQ ID NOs: 1, 48, 63, 66, 85, and 100, respectively;
(76) the heavy chain CDR1, the heavy chain CDR2, the heavy chain CDR3, the light chain CDR1, the light chain CDR2, and the light chain CDR3 are amino acid sequences shown in SEQ ID NOs: 1, 43, 63, 66, 73, and 106, respectively;
(77) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 88, and 100, respectively;
(78) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 89, and 100, respectively;
(79) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 90, and 100, respectively;
(80) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 92, and 100, respectively;
(81) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 91, and 100, respectively;
(82) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 94, and 100, respectively;
(83) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 93, and 100, respectively;
(84) the heavy chain CDR1, the heavy chain CDR2, the heavy chain CDR3, the light chain CDR1, the light chain CDR2, and the light chain CDR3 are amino acid sequences shown in SEQ ID NOs: 1, 42, 57, 4, 93, and 109, respectively;
(85) the heavy chain CDR1, the heavy chain CDR2, the heavy chain CDR3, the light chain CDR1, the light chain CDR2, and the light chain CDR3 are amino acid sequences shown in SEQ ID NOs: 23, 42, 57, 4, 73, and 100, respectively;
(86) the heavy chain CDR1, the heavy chain CDR2, the heavy chain CDR3, the light chain CDR1, the light chain CDR2, and the light chain CDR3 are amino acid sequences shown in SEQ ID NOs: 29, 42, 57, 4, 73, and 100, respectively;
(87) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 108, respectively;
(88) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 107, respectively;
(89) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 73, and 109, respectively;
(90) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 73, and 100, respectively;
(91) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 69, 73, and 100, respectively;
(92) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 73, and 109, respectively;
(93) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 93, and 109, respectively;
(94) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 65, 86, and 100, respectively;
(95) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 86, and 100, respectively;
(96) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 67, 73, and 108, respectively;
(97) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 95, and 108, respectively;
(98) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 57, 4, 94, and 108, respectively;
(99) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 100, respectively;
(100) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 73, and 100, respectively;
(101) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 68, 73, and 100, respectively;
(102) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 108, respectively;
(103) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 107, respectively;
(104) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 73, and 109, respectively;
(105) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 69, 73, and 100, respectively;
(106) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 73, and 109, respectively;
(107) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 86, and 100, respectively;
(108) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 4, 93, and 109, respectively;
(109) the heavy chain CDR1, the heavy chain CDR2, the heavy chain CDR3, the light chain CDR1, the light chain CDR2, and the light chain CDR3 are amino acid sequences shown in SEQ ID NOs: 27, 45, 57, 65, 86, and 100, respectively;
(110) the heavy chain CDR1, the heavy chain CDR2, the heavy chain CDR3, the light chain CDR1, the light chain CDR2, and the light chain CDR3 are amino acid sequences shown in SEQ ID NOs: 34, 42, 57, 65, 73, and 100, respectively;
(111) the heavy chain CDR1, the heavy chain CDR2, the heavy chain CDR3, the light chain CDR1, the light chain CDR2, and the light chain CDR3 are amino acid sequences shown in SEQ ID NOs: 1, 44, 57, 68, 73, and 100, respectively;
(112) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 65, 73, and 100, respectively;
(113) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 69, 73, and 100, respectively;
(114) the heavy chain CDR1, the heavy chain CDR2, the heavy chain CDR3, the light chain CDR1, the light chain CDR2, and the light chain CDR3 are amino acid sequences shown in SEQ ID NOs: 1, 45, 57, 4, 86, and 100, respectively;
(115) the heavy chain CDR1, the heavy chain CDR2, the heavy chain CDR3, the light chain CDR1, the light chain CDR2, and the light chain CDR3 are amino acid sequences shown in SEQ ID NOs: 28, 42, 57, 4, 73, and 108, respectively;
(116) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 59, 4, 73, and 108, respectively;
(117) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 58, 4, 73, and 108, respectively;
(118) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 60, 4, 73, and 108, respectively;
(119) the heavy chain CDR1, the heavy chain CDR2, the heavy chain CDR3, the light chain CDR1, the light chain CDR2, and the light chain CDR3 are amino acid sequences shown in SEQ ID NOs: 27, 42, 61, 4, 73, and 108, respectively.

4. The antibody or antigen-binding fragment thereof according to any one of claims 1-3, wherein a heavy chain variable region thereof has an amino acid sequence having at least 75%, 80%, 85%, 90%, 95%, 98%, 99%, or 100% identity to a sequence shown in SEQ ID NO: 7, and a light chain variable region thereof has an amino acid sequence having at least 75%, 80%, 85%, 90%, 95%, 98%, 99%, or 100% identity to a sequence shown in SEQ ID NO: 8.

5. The antibody or antigen-binding fragment thereof according to any one of claims 1-4, further comprising a heavy chain constant region shown in SEQ ID NO: 9 and a light chain constant region shown in SEQ ID NO: 10.

6. The antibody or antigen-binding fragment thereof according to any one of claims 1-5, wherein the antibody or antigen-binding fragment thereof comprises a single-chain antibody, a single-domain antibody, a diabody, a triabody, a disulfide-stabilized antibody, a nanobody, a Fab fragment, a Fab' fragment, a (Fab')₂ fragment, a minibody, a monoclonal antibody, a polyclonal antibody, a monospecific antibody, a bispecific antibody, a multispecific antibody, a monovalent antibody, a multivalent antibody, a chimeric antibody, a fusion protein comprising an antigen-binding site of an antibody, or a full-length antibody immunoglobulin IgG1, IgG2, IgG3, IgG4, IgA, IgE, IgM, or IgD.

7. The antibody or antigen-binding fragment thereof according to any one of claims 1-6, wherein a heavy chain thereof is an amino acid sequence obtained by mutation of at least 1, 2, 3, 4, 5, 6, 7, 8, 9, 10, 11, 12, 13, 14, 15, 16, or 17 positions occurring on a sequence shown in SEQ ID NO: 11, and the mutation comprises an insertion, deletion, and/or substitution.

8. The antibody or antigen-binding fragment thereof according to any one of claims 1-7, wherein the mutation is a substitution, and an amino acid position for the substitution is selected from at least one of positions 27, 29, 31, 32, 33, 34, 35, 54, 58, 59, 61, 62, 100, 103, 105, 106, and 108 of the sequence shown in SEQ ID NO: 11.

9. The antibody or antigen-binding fragment thereof according to any one of claims 1-8, wherein a light chain variable region thereof is an amino acid sequence obtained by mutation of at least 1, 2, 3, 4, 5, 6, 7, 8, 9, 10, 11, 12, 13, or 14 positions occurring on a sequence shown in SEQ ID NO: 12, and the mutation comprises an insertion, deletion, and/or substitution.

10. The antibody or antigen-binding fragment thereof according to any one of claims 1-9, wherein the mutation is a substitution, and an amino acid position for the substitution is selected from at least one of positions 28, 31, 32, 33, 49, 50, 51, 52, 54, 55, 88, 92, 94, and 95 of the sequence shown in SEQ ID NO: 12.

11. A polynucleotide, wherein the polynucleotide encodes the antibody or antigen-binding fragment thereof according to any one of claims 1-10.

12. A recombinant vector, wherein the recombinant vector comprises the polynucleotide according to claim 11.

13. A host cell, wherein the host cell comprises the polynucleotide according to claim 11 and/or the recombinant vector according to claim 12.

14. A pharmaceutical composition, wherein the pharmaceutical composition comprises the antibody or antigen-binding fragment thereof according to any one of claims 1-10.

15. A kit, wherein the kit comprises the antibody or antigen-binding fragment thereof according to any one of claims 1-10.

16. A multispecific antibody, wherein the multispecific antibody comprises the antibody or antigen-binding fragment thereof according to any one of claims 1-10, and further comprising one or more second antibodies or antigen-binding fragments thereof which bind specifically to other antigens.

17. The multispecific antibody according to claim 16, wherein the second antibody or the antigen-binding fragment thereof is selected from a full-length antibody, a Fab, a Fab", a (Fab")2, an Fv, an scFv, an scFv-scFv, a minibody, a diabody, or an sdAb.

18. An antibody conjugate, wherein the antibody conjugate comprises the antibody or antigen-binding fragment thereof according to any one of claims 1-10, and a second functional structure selected from an Fc, a radioisotope, a half-life extending structural moiety, a detectable label, and a drug.

19. The antibody conjugate according to claim 18, wherein
the half-life extending structural moiety is selected from an albumin-binding structure, a transferrin-binding structure, a polyethylene glycol molecule, a recombinant polyethylene glycol molecule, a human serum albumin, a human serum albumin fragment, and a human serum albumin-binding polypeptide;
the detectable label is selected from a fluorophore, a chemiluminescent compound, a bioluminescent compound, an enzyme, an antibiotic resistance gene, and a contrast agent; and/or
the drug is selected from a cytotoxin and an immunomodulator.

20. Use of the antibody or antigen-binding fragment thereof according to any one of claims 1-10, the multispecific antibody according to claim 16 or 17, and/or the antibody conjugate according to claim 18 or 19 in preparing a drug, a reagent, or a kit for detecting, preventing, alleviating, or treating a disease.

21. The use according to claim 20, wherein the disease is associated with elevated expression levels of oligomers, dimers, tetramers, or higher-order multimers of S100A8 and S100A9.

22. The use according to claim 20, wherein the disease comprises myocardial infarction and myocardial ischemia/reperfusion injury.

23. Use of the antibody or antigen-binding fragment thereof according to any one of claims 1-10, the multispecific antibody according to claim 16 or 17, and/or the antibody conjugate according to claim 18 or 19 in preparing a drug, a reagent, or a kit for reducing a myocardial infarct size, improving a cardiac function, or improving a microcirculatory function.

24. A method for detecting, preventing, alleviating, or treating a disease, wherein the method comprises administrating to a subject in need thereof the antibody or antigen-binding fragment thereof according to any one of claims 1-10, the pharmaceutical composition according to claim 14, the kit according to claim 15, the multispecific antibody according to claim 16 or 17, and/or the antibody conjugate according to claim 18 or 19.

25. The method according to claim 24, wherein the disease is associated with elevated expression levels of oligomers, dimers, tetramers, or higher-order multimers of S100A8 and S100A9.

26. The method according to claim 24, wherein the disease comprises ischemic organ injury and organ ischemia/reperfusion injury related to S100A8/A9, S100A8, or S100A9.

27. A method for reducing a myocardial infarct size, improving a cardiac function, or improving a microcirculatory function, wherein the method comprises administrating to a subject in need thereof the antibody or antigen-binding fragment thereof according to any one of claims 1-10, the pharmaceutical composition according to claim 14, the kit according to claim 15, the multispecific antibody according to claim 16 or 17, and/or the antibody conjugate according to claim 18 or 19.
